# EUROPEAN PATENT APPLICATION

(11) **EP 4 494 656 A1**
(43) Date of publication of application: **22.01.2025**
(21) Application number: 23187008.0
(22) Date of filing: 21.07.2023
(51) Int. Cl.: A61K 47/54, A61K 47/55, A61K 47/68, A61P 35/00

(54) **ORGANIC CYTOTOXIN OLIGOMERS AND USES THEREOF AS A PAYLOAD IN ANTIBODY-DRUG CONJUGATES**

(71) Applicant: Simris Biologics GmbH, 12489 Berlin (DE)
(72) Inventor: NIEDERMEYER, Timo, 06114 Halle (DE); SCHUSTER, Sabine, 06120 Halle (DE); ENKE, Heike, 12307 Berlin (DE); ENKE, Dan, 12307 Berlin (DE)
(74) Representative: Andresen, Heiko

(57) **Abstract**

A pharmaceutical cytotoxin oligomer is provided. The pharmaceutical cytotoxin oligomer comprises a first organic cytotoxin entity, at least a second organic cytotoxin entity, and a crosslinking entity forming a covalent linkage between the first organic cytotoxin entity and the second organic cytotoxin entity, wherein a monomer of the first organic cytotoxin entity and a monomer of the second organic cytotoxin entity each exerts a cytotoxic effect on a target cell by being actively transported into the target cell by a transporter protein of the target cell, and/or by inhibiting an intracellular enzyme of the target cell. The covalent linkage between the first organic cytotoxin entity and the second organic cytotoxin entity is substantially resistant to cleavage under physiological conditions.

## Description

### Sequence Listing

This application contains an electronic sequence listing in xml-format according to WIPO ST.26 standard with 16 sequences as part of the description, the content of which is hereby incorporated by reference in its entirety.

### Field of invention

This invention relates to biologically active cytotoxic anticancer payloads or drugs. In particular, this invention relates to organic cytotoxins, more particularly engineered cyanobacterial and fungal toxins, for use as a payload in an antibody-drug conjugate. This invention further relates to antibody-drug conjugates comprising such biologically active cytotoxic anticancer payloads coupled to an antibody that specifically targets a certain tumor antigen.

### Background of invention

Today, cancer is the second leading cause of death globally, accounting for about 10 million deaths per year worldwide. Due to global population growth and aging, cancer incidence rates have increased over the past decades and are expected to continue to rise.

Most anticancer drugs currently used in conventional chemotherapy have the disadvantage that they are generally cytotoxic, as they insufficiently discriminate between cancerous cells and healthy tissues, with known severe side effects and significant limitations on the quality of life of patients.

The need for anticancer drugs with a specific spectrum of action against malignant cells has driven the development of antibody-drug conjugates.

Antibody-drug conjugates, in the following also referred to as "ADCs", are a class of biopharmaceutical drugs designed as a targeted therapy for treating cancer. Unlike chemotherapy, ADCs are intended to target and kill tumor cells while sparing healthy cells. ADCs are biopharmaceuticals essentially composed of a monoclonal antibody linked to a biologically active and often cytotoxic drug moiety, herein also referred to as the "payload", thereby combining the target specificity of a monoclonal antibody with the cancer-killing ability of a cytotoxic drug.

Conceptually, ADCs are supposed to target tumor cells and spare healthy tissues, mediated by the target specificity of the monoclonal antibody. Nevertheless, off-target effects are often seen during clinical application, caused for example by spontaneous or enzymatic hydrolysis of the chemical linkage between drug and antibody during transport and metabolism after systemic administration, or by the release of the payload from lysing or killed cancer cells into the surrounding tissue.

Current efforts to reduce or eliminate the undesirable toxic side effects on healthy cells are mainly directed to improving the stability of the chemical bonds between the cytotoxic payload and the monoclonal antibody in order to avoid premature release of the drug outside the tumor cell.

For example, WO 2018/206715 A2 and WO 2018/219619 A1, the disclosures of which are hereby incorporated by reference in their entirety, disclose modified cyanobacterial microcystins and nodularins as cytotoxic payloads which incorporate, among others, non-naturally occurring amino acids providing a chemical anchor group for the coupling of the payload to a linker or an antibody, respectively, thereby leading to homogeneous and stably loaded ADCs with limited payload release in the blood stream.

However, an improved chemical linkage between the antibody and the cytotoxic payload can only partially overcome the above-noted drawbacks of currents ADCs. In particular, it does not solve the problem of payload release after the targeted cancer cells died. Furthermore, the high potency of the cytotoxins creates unresolved health and safety risks in their manufacture and handling, resulting in cost-intensive infrastructure and production.

The hitherto unpublished European Patent Application No. 22192517.5, the disclosure of which is hereby incorporated by reference in its entirety, teaches modifying cyanobacterial or fungal toxins with structural modifications comprising an organic inhibitory group or effector molecule reducing the uptake of the modified toxin by transporter proteins into the target cell.

In this regard, it is an object of the present invention to provide further improved organic cytotoxins for use as a payload in an antibody-drug conjugate. Another object of the present invention is to provide an improved antibody-drug conjugate.

These objectives are solved in accordance with the subject matters of the independent claims. Preferred embodiments are subject matters of the dependent claims and the following description.

### Summary of invention

In certain embodiments, the invention provides a pharmaceutical cytotoxin oligomer or a pharmaceutically acceptable salt thereof, the pharmaceutical cytotoxin oligomer comprising a first organic cytotoxin entity, at least a second organic cytotoxin entity, and a crosslinking entity forming a covalent linkage between the first organic cytotoxin entity and at least the second organic cytotoxin entity, wherein a monomer of each of the first organic cytotoxin entity and the second organic cytotoxin entity exerts a cytotoxic effect on a target cell i) by being actively transported into the target cell by a transporter protein of the target cell, and/or ii) by inhibiting an intracellular enzyme of the target cell, wherein the covalent linkage between the first organic cytotoxin entity and at least the second organic cytotoxin entity is substantially resistant to cleavage under physiological conditions.

In certain embodiments, the invention provides a method for manufacturing a pharmaceutical cytotoxin oligomer as defined above, comprising A) providing a first organic cytotoxin entity and at least a second cytotoxin entity, wherein each of the first organic cytotoxin entity and the second organic cytotoxin entity exerts a cytotoxic effect on a target cell i) by being actively transported into the target cell by a transporter protein of the target cell, and/or ii) by inhibiting an intracellular enzyme of the target cell, B) forming a covalent linkage between the first organic cytotoxin entity and the second organic cytotoxin entity that is substantially resistant to cleavage under physiological conditions, thereby forming the pharmaceutical cytotoxin oligomer.

In certain embodiments, the invention provides a use of a pharmaceutical cytotoxin oligomer as defined above for manufacturing an antibody-drug conjugate comprising covalently conjugating the pharmaceutical cytotoxin oligomer to an antibody or an antigen-binding fragment thereof.

In certain embodiments, the invention provides an antibody-drug conjugate comprising an antibody or an antigen-binding fragment thereof, and a pharmaceutical cytotoxin oligomer or a pharmaceutically acceptable salt thereof as defined above, wherein the pharmaceutical cytotoxin oligomer is covalently attached to the antibody or the antigen-binding fragment thereof.

In certain embodiments, the invention provides a method for manufacturing an antibody-drug conjugate, comprising a) providing a pharmaceutical cytotoxin oligomer or a pharmaceutically acceptable salt thereof as defined above, b) providing an antibody or an antigen-binding fragment thereof, c) covalently attaching the pharmaceutical cytotoxin oligomer to the antibody or the antigen-binding fragment thereof.

In certain embodiments, the invention provides a pharmaceutical cytotoxin oligomer or a pharmaceutically acceptable salt thereof as defined above or an antibody-drug conjugate as defined above for use in the treatment of a malignant disease, in particular cancer, more particularly a malignant tumor.

In certain embodiments, the invention provides a method for treating a malignant disease in a subject, comprising administering to the subject a pharmaceutically effective amount of a pharmaceutical cytotoxin oligomer or a pharmaceutically acceptable salt thereof as defined above or a pharmaceutically effective amount of an antibody-drug conjugate as defined above.

In some embodiments according to, or as applied to, any of the embodiments above, the first organic cytotoxin entity and the second organic cytotoxin entity, independently of each other, is selected from a group consisting of a cyanobacterial toxin, a fungal toxin, or a variant or derivative thereof.

In some embodiments according to, or as applied to, any of the embodiments above, the first organic cytotoxin entity and the second organic cytotoxin entity each comprises a cyclic oligopeptide with a plurality of amino acids, wherein the crosslinking entity covalently links a side-chain of one of the amino acids of the first organic cytotoxin entity with a side-chain of one of the amino acids of the second organic cytotoxin entity.

In some embodiments according to, or as applied to, any of the embodiments above, the intracellular enzyme of the target cell is a phosphatase or a polymerase.

In some embodiments according to, or as applied to, any of the embodiments above, the resistance to cleavage comprises resistance to lysosomal degradation.

Preferred variants of these embodiments can be derived from the description of the invention and the detailed description of embodiments.

### Description of invention

The invention is based on the inventors' insight that cytotoxins generally must be taken up by cells either through active transport mediated by transport proteins or by passive diffusion before they can exert their cytotoxic function. However, when cytotoxins are coupled to an antibody, thereby forming an ADC, the internalization of the cytotoxins changes to receptor-mediated endocytosis, e. g. clathrin-mediated endocytosis, caveolae-mediated endocytosis or pinocytosis, a process by which the cells absorb the ADC by inward budding of the plasma membrane after binding of the antibody to the cell surface, thereby transporting the toxins into the targeted cells.

Based on this insight, the inventors describe in European Patent Application No. 22192517.5 the potential of modifying organic cytotoxins which, in an isolated form, require active transport across a cell membrane and cannot enter a cell by passive diffusion, with a structural modification that deliberately impairs their acceptance by transporter proteins. In this way, the inventors equip organic cytotoxins with a structural "safety catch" that prevents them from being actively taken up into cells and exerting their cytotoxic function, thereby protecting healthy cells from cytotoxic side-effects.

In the course of further intensive research, the inventors have now discovered that a particularly advantageous structural modification to this effect is artificial oligomerization of organic cytotoxins via a non-cleavable covalent linkage. Firstly, the inventors found that the artificial oligomerization strongly inhibits active cellular uptake of organic cytotoxins. Secondly, the inventors observed a surprising improvement in cytotoxicity of the oligomerized cytotoxins in a synergistic mode of action, wherein the cytotoxicity of the oligomerized cytotoxins significantly exceeds the aggregated cytotoxicity of the monomeric cytotoxin equivalents.

Due to the cumulative pharmacological interplay of both effects, the cytotoxin oligomers of the present invention expand the therapeutic window, i. e. the range of doses optimized between efficacy and toxicity, by several orders of magnitude in comparison to the monomeric cytotoxin counterparts, achieving greater therapeutic benefit without resulting in unacceptable side-effects or toxicity. In this respect, reference is also made to the detailed description of embodiments.

Against this background, one aspect of the present invention relates to a pharmaceutical cytotoxin oligomer or a pharmaceutically acceptable salt thereof. In particular, the present invention relates to a drug moiety, also referred to as "payload", comprising, or consisting of, a pharmaceutical cytotoxin oligomer or a pharmaceutically acceptable salt thereof.

The pharmaceutical cytotoxin oligomer of the present invention comprises a first organic cytotoxin entity, at least a second organic cytotoxin entity, and a crosslinking entity forming a covalent linkage between the first organic cytotoxin entity and at least the second organic cytotoxin entity.

The expressions "first" and "second" and, as the case may be, "third", "fourth" and so on, are used herein to distinguish one feature or element of the invention from another, but should otherwise not be considered limiting unless the context indicates otherwise. Accordingly, a "first organic cytotoxin entity" described hereunder could be referred to as a "second organic cytotoxin entity", and correspondingly, a "second organic cytotoxin entity" described hereunder could be referred to as a "first organic cytotoxin entity" without departing from the teachings of the present invention.

In order to achieve the advantageous effect according to the present invention, it is essential that each of the first organic cytotoxin entity and the second organic cytotoxin entity, when in a monomeric form, i) requires active transport into a target cell by a transporter protein to exert its cytotoxic effect on the target cell, and, alternatively or in addition, ii) is an enzyme inhibitor, i. e. exerts its cytotoxic effect by inhibition of an intracellular enzyme of a target cell, which is preferably an intracellular enzyme whose bioactivity is essential for the target cell so that its inhibition is cytotoxic and leads to death of the target cell.

In addition, an essential feature of the invention in this respect is that the covalent linkage between the first organic cytotoxin entity and at least the second organic cytotoxin entity is substantially resistant to cleavage under physiological conditions.

As used herein, the term "physiological conditions" refers to conditions of the internal body milieu that occurs in nature e. g. in the human bloodstream, the intracellular environment and/or extracellular environment, as opposed to artificial laboratory conditions. In particular, physiological conditions include a temperature range of 20-40 degrees Celsius, atmospheric pressure of about 1 bar, pH of 6-8, glucose concentration of 1-20 mM, and atmospheric oxygen concentration.

The term "substantially resistant" means that spontaneous cleavage such as spontaneous hydrolysis of the covalent linkage is extremely slow, with the half-life of the covalent linkage at 25 °C being typically several months or several years, in certain embodiments several decades. In certain embodiments, the term "substantially resistant" means resistance to lysosomal degradation. In certain embodiments, "substantially resistant" means resistance to enzymatic and/or chemical cleavage, such as resistance to hydrolase and/or oxidoreductase cleavage, and/or resistance to acid-induced cleavage at least down to a pH value of pH 4.5 or pH 4.0.

In this way, the cytotoxin oligomers of the present invention, as opposed to the corresponding cytotoxin monomers, are blocked from unfolding unspecific cytotoxic activity in an extracellular environment in the human body, e. g. upon premature release from the ADC into the bloodstream or after release from a dead cancer cell. In contrast, when the cytotoxin oligomers of the present invention are bound to an antibody, they are internalized by target cells as the antibody's payload through receptor-mediated endocytosis, where, after intracellular trafficking and lysosomal degradation of the ADC, the cytotoxin oligomers are released and the enhanced cytotoxicity of the cytotoxin oligomers is unleashed to trigger tumor cell death. In this way, the present invention provides cytotoxin oligomers for use as ADC payloads with significantly enhanced safety profile, which allows to increase the dosage of the ADC for improved therapeutic benefit without resulting in unacceptable side-effects or toxicity. In addition, safety during production and processing of the cytotoxin oligomers is improved in comparison to the monomeric cytotoxin counterparts. It is understood that the beneficial effect is not limited to ADCs, but is also achieved when the cytotoxin oligomers are coupled to other targeting moieties, i. e. molecules or macromolecules that bind to specific receptors located on the surface of tumoral cells, including, but not limited to, nanoparticles, liposomes, micelles, aptamers, peptides and dendrimers.

In the sense of the present invention, the term "oligomer" or "oligomeric" refers to a molecule that is derived from connecting smaller molecules, the "monomers", by covalent bonds.

Accordingly, the term "organic cytotoxin entity" refers to a cytotoxin moiety derived from a cytotoxin monomer having an organic base structure known to be cytotoxic for a target cell, in particular a cancer cell, after uptake by a transporter protein of the target cell. Each organic cytotoxin entity of a cytotoxin oligomer may also be referred to as a "cytotoxin subunit". Hereinafter, a cytotoxin oligomer composed of two organic cytotoxin entities is referred to as "dimer", a cytotoxin oligomer composed of three organic cytotoxin entities as "trimer", a cytotoxin oligomer composed of four organic cytotoxin entities as "tetramer", and so forth. In preferred embodiments, the cytotoxin oligomer is a dimer, a trimer or a tetramer, more preferably a dimer or a trimer, most preferred a dimer. If the cytotoxin oligomer is composed of organic cytotoxin entities that are identical, the cytotoxin oligomer is referred to as a homooligomer; otherwise the cytotoxin oligomer is referred to as a heterooligomer.

Cytotoxin monomers with an organic base structure that requires a transporter protein for active cell uptake of the toxin are known to the skilled artisan. For example, transport of the cytotoxin monomers or the organic base structure, respectively, can be mediated by an organic anion transporting polypeptide (OATP) or a subtype thereof, such as OATP1B1, OATP1B3 and/or OATP1A2. These OATPs are primarily found in the liver of humans. Therefore, the inventors have realized the importance of blocking transport of payload toxins by OATPs to circumvent hepatotoxicity and improve the relative safety of the ADC.

In preferred embodiments of the invention, the first organic cytotoxin entity and the second organic cytotoxin entity, independently of each other, is selected from a group consisting of a cyanobacterial toxin, a fungal toxin, or a variant or derivative thereof. In accordance with the usual understanding in the art, the term "derivative" refers to organic cytotoxin entities having the same base structure as a cyanobacterial toxin or a fungal toxin but having a functional group or another atom or atomic group instead of a hydrogen atom or substituent, or wherein one or more atoms or atomic groups of the base structure were removed. Accordingly, the term "variant" refers to organic cytotoxin entities having a partially altered but therapeutically equivalent base structure of a cyanobacterial toxin or fungal toxin.

In further preferred embodiments of the invention, the first organic cytotoxin entity and the second organic cytotoxin entity each comprises a cyclic oligopeptide with a plurality of amino acids, wherein the crosslinking entity covalently links a side-chain of one of the amino acids of the first organic cytotoxin entity with a side-chain of one of the amino acids of the second organic cytotoxin entity. In preferred embodiments, the cyclic oligopeptide comprises from 4 to 10 amino acids, in particular 5 to 8 amino acids, e. g. exactly four, exactly five, exactly six, exactly seven or exactly eight amino acids. Preferably, the cyclic oligopeptide toxin comprises at least one non-proteinogenic amino acid, preferably several non-proteinogenic amino acids. More preferably, the cyclic oligopeptide includes a dipeptide sequence with a first amino acid selected from the group consisting of Adda, DM-Adda, dm-Adda, (6Z)Adda and ADM-Adda, and a second amino acid selected from the group consisting of D-Glu and D-Glu(OCH₃). More preferably, the first amino acid is selected from the group consisting of Adda, DM-Adda, dm-Adda and ADM-Adda, and the second amino acid is D-Glu.

In certain embodiments, the first organic cytotoxin entity and/or the second organic cytotoxin entity is an enzyme inhibitor, in particular a protein phosphatase inhibitor or a polymerase inhibitor, such as an RNA polymerase inhibitor.

In preferred embodiments of the invention, the cyanobacterial toxin is a microcystin. Microcystins are cyanobacterial cyclic heptapeptides that potently inhibit eukaryotic serine/threonine protein phosphatases type 1 and 2A, leading to an interruption of many essential signal transduction pathways and finally to the collapse of the cytoskeleton and the death of the cell. Microcystins are primarily taken up by OATP1B1 and OATP1B3.

In other preferred embodiments of the invention, the cyanobacterial toxin is a nodularin. Nodularins are cyanobacterial cyclic pentapeptides evolutionary related to microcystins that also inhibit protein phosphatases type 1 and 2A as their cytotoxic mode of action. Nodularins that are primarily taken up by OATP1B1.

In yet other preferred embodiments of the invention, the fungal toxin is an amanitin. Amanitins are fungal bicyclic octapeptides belonging to the subgroup of amatoxins. The most prominent alpha-Amanitin is an inhibitor of RNA polymerase II, binding to the bridging helix of RNA polymerase II and inhibiting the translocation of RNA and DNA needed to empty the site for the next synthesis run.

In preferred embodiments of the invention, the first organic cytotoxin entity and the second organic cytotoxin entity each comprises a microcystin having a general structure

cyclo (-Aa¹-X²-Aa³-Z⁴-Aa⁵-Aa⁶-Aa⁷).

Herein, independently from each other, Aa¹ and Aa³ each represents a D-amino acid which may be modified, Aa⁵ is selected from the group consisting of Adda, DM-Adda, dm-Adda, (6Z)Adda, and ADM-Adda, Aa⁶ is selected from the group consisting of D-Glu and D-Glu(OCH₃), Aa⁷ is selected from the group consisting of Mdha, MdhB, Dha, L-Ser, L-MeSer, Dhb, (E)-Dhb, (Z)-Dhb, MeLan, Cys and Thr or a modified L-amino acid, and X² and Z⁴ are, independently from each other, an L-amino acid which may be modified. Mdha is N-methyldehydroalanine, Mdhb is N-methyldehydrobutyrate. Adda is 3-amino-9-methoxy-2,6,8-trimethyl-10-phenyl-deca-4,6-dienoic acid, ADM-Adda is O-acetyl-9-0-desmethyl-Adda, DM-Adda is 9-0-desmethyl-Adda, dm-Adda is Adda demethylated at C-2, C-6, or C-8, Dha is dehydroalanine, MeSer is N-methyl-L-serine, Dhb is dehydrobutyrate, MeLan is N-methyl-lanthionin. As used herein, a "modified" or "modified amino acid" means an amino acid with a side-chain having a site of attachment to the crosslinking entity, thereby adjoining the crosslinking entity. In particular, the side-chain of the modified amino acid can comprise a chemical group incorporated in, or forming part of, the covalent linkage between the first and second cytotoxin entity.

In some embodiments, Aa¹ is selected from the group consisting of D-Ala, D-Leu, D-Ser, Gly or a modified D-amino acid or a modified Gly. In some embodiments, Aa³ is selected from the group consisting of D-Asp and D-MeAsp or a modified D-amino acid. D-MeAsp is D-erythro-β-methylaspartic acid. In some embodiments, Aa⁵ is selected from the group consisting of Adda, DM-Adda, dm-Adda and ADM-Adda. In some embodiments, Aa⁶ is D-Glu. The modified amino acid may for example be derived from, i. e. a derivative of, an amino acid not present in a corresponding natural cyanobacterial or fungal toxin having side-chain with a coupling functionality. As used herein, a "functionality" is the presence of one or more functional groups in a molecule. A monofunctional molecule possesses one functional group, a bifunctional or difunctional molecule two functional groups, a trifunctional molecule three functional groups, and so forth. In organic chemistry, a functionality has a decisive influence on the reactivity of a molecule. Accordingly, a "coupling functionality" is a functional group capable of, and/or configured for, forming at least part of a covalent linkage with another coupling functionality having complementary reactivity. For example, the modified amino acid may be derived from the group consisting of azidonorvaline, propargyltyrosine, azidolysine, azidophenylalanine, propargylcysteine, propargylserine and azidoalanine, either as D-amino acid or L-amino acid as applicable.

Microcystins are potent inhibitors of type 1 and type 2A protein phosphatases. Protein phosphatases 1 (PP1) and 2A (PP2A) are two of the major phosphatases in eukaryotic cells that dephosphorylate serine and threonine residues. For example, the IC50 of naturally occurring microcystin-LR (MC-LR, SEQ ID NO:16) is 0.03 nM for type 1 protein phosphatase and 0.04 nM for type 2A protein phosphatase. It is established in the art that positions A⁵ and A⁶ are essential and sufficient for the inhibitory effect of microcystins against PP1 and PP2A.

Therefore, in preferred embodiments, the crosslinking entity covalently links a side-chain of one of Aa¹, X², Aa³, Z⁴ or Aa⁷ of the first organic cytotoxin entity with a side-chain of one of Aa¹, X², Aa³, Z⁴ or Aa⁷ of the second organic cytotoxin entity.

In some embodiments, the crosslinking entity covalently links a side-chain of one of Aa¹, X², Z⁴ or Aa⁷ of the first organic cytotoxin entity with a side-chain of one of Aa¹, X², Z⁴ or Aa⁷ of the second organic cytotoxin entity.

In some embodiments, the crosslinking entity covalently links a side-chain of one of X², Z⁴ or Aa⁷ of the first organic cytotoxin entity with a side-chain of one of X², Z⁴ or Aa⁷ of the second organic cytotoxin entity.

In some embodiments, the crosslinking entity covalently links a sidechain of one of X² or Z⁴ of the first organic cytotoxin entity with a side-chain of one of X² or Z⁴ of the second organic cytotoxin entity.

In some embodiments, the crosslinking entity covalently links a sidechain of Aa¹ of the first organic cytotoxin entity with a side-chain of one of Aa¹, X², Aa³, Z⁴ or Aa⁷ of the second organic cytotoxin entity. In some embodiments, the crosslinking entity covalently links a sidechain of X² of the first organic cytotoxin entity with a side-chain of one of Aa¹, X², Aa³, Z⁴ or Aa⁷ of the second organic cytotoxin entity. In some embodiments, the crosslinking entity covalently links a sidechain of Aa³ of the first organic cytotoxin entity with a side-chain of one of Aa¹, X², Aa³, Z⁴ or Aa⁷ of the second organic cytotoxin entity. In some embodiments, the crosslinking entity covalently links a sidechain of Z⁴ of the first organic cytotoxin entity with a side-chain of one of Aa¹, X², Aa³, Z⁴ or Aa⁷ of the second organic cytotoxin entity. In some embodiments, the crosslinking entity covalently links a sidechain of Aa⁷ of the first organic cytotoxin entity with a side-chain of one of Aa¹, X², Aa³, Z⁴ or Aa⁷ of the second organic cytotoxin entity.

In other preferred embodiments of the invention, the first organic cytotoxin entity and the second organic cytotoxin entity each comprises a nodularin having a general structure

cyclo [-Aa¹-Aa²-Aa³-Aa⁴-Aa⁵].

Herein, independently from each other, Aa¹ is D-Asp or D-MeAsp or a modified D-amino acid, Aa² is Arg or Har or a modified L-amino acid, Aa³ is selected from the group consisting of Adda, DM-Adda, (6Z)Adda, and MeAdda, Aa⁴ is selected from the group consisting of D-Glu and D-Glu(OCH₃), Aa⁵ is Dhb or Mdhb or a modified L-amino acid. Har is homoarginine, (6Z)Adda is a geometrically isomer of Adda at C-6, MeAdda is Adda methylated.

It is established in the art that positions A³ an A⁴ are essential and sufficient for the inhibitory effect of nodularins against PP1 and PP2A. Therefore, in preferred embodiments, the crosslinking entity covalently links a side-chain of Aa¹, Aa² or Aa⁵ of the first organic cytotoxin entity with a side-chain of one of Aa¹, Aa² or Aa⁵ of the second organic cytotoxin entity.

In some embodiments, the crosslinking entity covalently links a side-chain of one of Aa¹ or Aa⁵ of the first organic cytotoxin entity with a side-chain of one of Aa¹ or Aa⁵ of the second organic cytotoxin entity.

In some embodiments, the crosslinking entity covalently links a sidechain of Aa¹ of the first organic cytotoxin entity with a side-chain of one of Aa¹, Aa² or Aa⁵ of the second organic cytotoxin entity. In some embodiments, the crosslinking entity covalently links a sidechain of Aa² of the first organic cytotoxin entity with a side-chain of one of Aa¹, Aa² or Aa⁵ of the second organic cytotoxin entity. In some embodiments, the crosslinking entity covalently links a sidechain of Aa⁵ of the first organic cytotoxin entity with a side-chain of one of Aa¹, Aa² or Aa⁵ of the second organic cytotoxin entity.

In yet other preferred embodiments, the first organic cytotoxin unit and the second organic cytotoxin unit each comprises an amatoxin having a general structure
cyclo{Aa¹-cyclo[Aa²-Aa³-Aa⁴-Aa⁵-Aa⁶]-Aa⁷-Aa⁸} (SEQ ID NO:1).

Herein, independently from each other, Aa¹ is Ile, hydroxyisoleucine (Hil) or dihydroxyisoleucine (Dhil) or a derivative thereof with a side-chain having a site of attachment to the crosslinking entity, Aa² is Trp or hydroxytryptophan (Htp) or a derivative thereof with a side-chain having a site of attachment to the crosslinking entity, Aa³ is Gly, Aa⁴ is Ile, Aa⁵ is Gly, Aa⁶ is Cys, Aa⁷ is Asn or Asp and Aa⁸ is Pro or hydroxyproline (Hyp) or a derivative thereof with a side-chain having a site of attachment to the crosslinking entity, with crosslinking between Aa² and Aa⁶ via a sulfoxide (S=O) bridge.

For example, the amatoxin can be one of the group consisting of α-amanitin, β-amanitin, γ-amanitin, δ-amanitin, ε-amanitin, amanullin, amanullinic acid, amaninamide, amanin and proamanullin. Preferably, the amatoxin is an amanitin.

In preferred embodiments, the crosslinking entity covalently links a side-chain of one of Aa¹, Aa² or Aa⁸ of the first organic cytotoxin entity with a side-chain of one of Aa¹, Aa² or Aa⁸ of the second organic cytotoxin entity.

In some embodiments, the crosslinking entity covalently links a side-chain of one of Aa² or Aa⁸ of the first organic cytotoxin entity with a side-chain of one of Aa² or Aa⁸ of the second organic cytotoxin entity.

In some embodiments, the crosslinking entity covalently links a side-chain of Aa¹ of the first organic cytotoxin entity with a side-chain of one of Aa¹, Aa² or Aa⁸ of the second organic cytotoxin entity. In some embodiments, the crosslinking entity covalently links a side-chain of Aa² of the first organic cytotoxin entity with a side-chain of one of Aa¹, Aa² or Aa⁸ of the second organic cytotoxin entity. In some embodiments, the crosslinking entity covalently links a side-chain of Aa⁸ of the first organic cytotoxin entity with a side-chain of one of Aa¹, Aa² or Aa⁸ of the second organic cytotoxin entity.

In some embodiments according to, or as applied to, any of the embodiments above, the cytotoxin oligomer comprises at least two crosslinking entities, each forming a covalent linkage between the first organic cytotoxin entity and the second organic cytotoxin entity in the above-described manner.

In some embodiments according to, or as applied to, any of the embodiments above, the crosslinking entity covalently links a side-chain in one amino acid position of the first organic cytotoxin entity with a side-chain in the same amino acid position of the second organic cytotoxin entity, e. g. Aa¹ with Aa¹, X² with X², Aa² with Aa², Aa³ with Aa³, Z⁴ with Z⁴, Aa⁷ with Aa⁷, Aa⁸ with Aa⁸, and so forth, as applicable.

In some embodiments according to, or as applied to, any of the embodiments above, the crosslinking entity covalently links a side-chain in one amino acid position of the first organic cytotoxin moiety with a side-chain in a different amino acid position of the second organic cytotoxin moiety, e. g. the side-chain of Aa² or X² of the first organic cytotoxin entity with the side-chain of any one of Aa¹, Aa³, Z⁴ or Aa⁷ of the second organic cytotoxin entity, the side-chain of Z⁴ of the first organic cytotoxin entity with a side-chain of any one of Aa¹, Aa² or X², Aa³ or Aa⁷ of the second organic cytotoxin entity, and the like.

In some embodiments according to, or as applied to, any of the embodiments above, the first organic cytotoxin entity and the second organic cytotoxin entity are substantially identical, i. e. the cytotoxin oligomer is a homooligomer, in particular a homodimer or a homotrimer. In certain embodiments, "substantially identical" means that the organic cytotoxin entities comprise, or are composed of, the same amino acid sequence. For example, the first cytotoxin entity and the second cytotoxin entity are derived from the same organic cytotoxin monomer.

In other embodiments according to, or as applied to, any of the embodiments above, the first organic cytotoxin entity and the second organic cytotoxin entity are different from each other, i. e. the cytotoxin oligomer is a heterooligomer, in particular a heterodimer or a heterotrimer. For example, the first organic cytotoxin entity and the second organic cytotoxin entity can comprise, or be composed of, a different amino acid sequence, e. g. including differently modified amino acids. In particular, the first cytotoxin entity and the second cytotoxin entity are derived from different organic cytotoxin monomers.

In some embodiments according to, or as applied to, any of the embodiments above, the crosslinking entity or the covalent linkage, respectively, comprises, or consists of, a covalent connection selected from the group consisting of an amide bond, a thioamide bond, an ether bond, a thioether bond, a covalent bond containing a triazole, a dihydropyridazine or a higher order amine such as a secondary (2°) amine, a tertiary (3°) amine or a quarternary (4⁰) amine, a carbamate bond, a thiocarbamate bond, an urea bond, a thiourea bond, a phosphate ester bond, a phosphamide bond, a sulfonamide bond, an oxime bond, or any combination thereof. The inventors have found that these types of covalent connections impart a resistance of the crosslinking entity to cleavage under the physiological conditions that enhances the advantageous pharmacological effect of the cytotoxin oligomer according to the invention. In certain embodiments, the crosslinking entity is free of disulfide bonds, ester bonds and/or isourea bonds. Moreover, the crosslinking entity is preferentially free of substrates for intracellular enzymatic cleavage, e. g. a hydrolase and/or oxidoreductase substrate or cleavage site, or a substrate for chemically triggered cleavage, e. g. in response to change of pH or change of reduction potential.

In some embodiments according to, or as applied to, any of the embodiments above, the crosslinking entity or the covalent linkage, respectively, comprises an organic bridging moiety that covalently interconnects the first organic cytotoxin entity and the second organic cytotoxin entity with each other. The organic bridging moiety can also spatially separate the first and second organic cytotoxin entity from each other. The organic bridging moiety is not particularly limited as long as the principal requirement of the invention that the covalent linkage between the first organic cytotoxin entity and the second organic cytotoxin entity is substantially resistant to cleavage under physiological conditions is fulfilled. Suitable organic bridging moieties are well known to the skilled artisan or can be determined on the basis of the present disclosure.

The organic bridging moiety can be linear, i. e. non-branched, or branched. A branched organic spacer unit may have three or more branches, wherein at least one of the branches is terminally connected to the first organic cytotoxin entity and a second of the branches is terminally connected to the second organic cytotoxin entity. A third or further branch can be terminally connected to a third or further organic cytotoxin entity and/or can have a terminal end comprising a coupling functionality, i. e. a functional group configured for covalent coupling of the cytotoxin oligomer to an antibody, optionally via a linker. The coupling functionality can optionally be protected with a protecting group.

Preferably, the bridging moiety is an organic molecule with a molecular size of from about 50 Da to about 3,000 Da, more preferably from about 50 Da to about 2,500 Da or from about 50 Da to about 2,000 Da, most preferred from about 50 Da to about 1,500 Da or from about 50 Da to about 1,000 Da. The bridging moiety can comprise methylene groups, PEG groups or other groups for spatial separation of the organic cytotoxin entities.

In some embodiments, at least one of the first organic cytotoxin entity, the second organic cytotoxin entity and the crosslinking entity comprises an extra coupling functionality, i. e. a functional group, configured for, i. e. capable of, covalent conjugation of the cytotoxin oligomer to an antibody, optionally via a linker. Suitable coupling functionalities for bioconjugation of the cytotoxin oligomer to an antibody are well known to the skilled artisan. For example, the extra coupling functionality can be a functional group selected from the group consisting of amino group, carboxyl group, hydroxyl group, azido group, alkyne group, alkene group, thiol group, aldehyde group, keto group, tetrazine group, hydrazine group or any combination thereof. The extra coupling functionality may be protected with a removable protecting group, including, but not limited to, a tert-Butyloxycarbonyl (BOC) group, an Acetyl (Ac) group, a tert-butyl ether (tBu), a methyl ester, a benzyl ester or a tert-butyl esters. Further suitable protecting groups are known to the skilled artisan. In preferred embodiments, the extra coupling functionality is configured for, or capable of, a bioorthogonal chemical coupling reaction such as a click chemistry reaction. A click chemistry reaction in the sense of the present invention is for example Copper(I)-catalyzed azide-alkyne cycloaddition, strain-promoted azide-alkyne cycloaddition, strain-promoted alkyne-nitrone cycloaddition, alkene and azide [3+2] cycloaddition, alkene and tetrazine inverse-demand Diels-Alder reaction, alkene and tetrazole photoclick reaction. Accordingly, the extra coupling functionality is preferably an azide, an alkyne, an alkene, or a tetrazine.

As already noted above, the cytotoxin oligomer can comprise more than two organic cytotoxin entities, forming a trimer, tetramer or even higher oligomer. For example, the cytotoxin oligomer can comprise at least a further third organic cytotoxin entity, wherein the crosslinking entity forms a covalent linkage between the first organic cytotoxin entity, the second organic cytotoxin entity and at least the further third organic cytotoxin entity, e. g. via a branched bridging moiety incorporated in the crosslinking entity as described above. Alternatively, the cytotoxin oligomer can comprise a first crosslinking entity forming a covalent linkage between the first organic cytotoxin entity and the second cytotoxin entity, and a second crosslinking entity forming a covalent linkage between the second cytotoxin entity and the third cytotoxin entity, wherein the covalent linkage between the first organic cytotoxin entity, the second organic cytotoxin entity and at least the further third organic cytotoxin entity in each case is substantially resistant to cleavage under physiological conditions.

In some embodiments according to, or as applied to, any of the embodiments herein, the resistance to cleavage comprises resistance to lysosomal degradation.

In preferred embodiments according to, or as applied to, any of the embodiments herein, the resistance to cleavage comprises resistance to enzymatic cleavage and resistance to acid-induced cleavage at least down to a pH value of pH 4.5 or pH 4.

The pharmaceutical cytotoxin oligomer described above can be advantageously used as a cytotoxic drug moiety ("payload") in an antibody-drug conjugate.

In some embodiments, the organic cytotoxin oligomer is selected from the group consisting of the organic cytotoxin oligomers described in the detailed description of embodiments below.

Another aspect of the present invention therefore relates to a use of a pharmaceutical cytotoxin oligomer as described above for manufacturing an antibody-drug conjugate. In particular, the use comprises covalently conjugating the pharmaceutical cytotoxin oligomer to an antibody or an antigen-binding fragment thereof.

In another aspect, the present invention provides an antibody-drug conjugate comprising an antibody or an antigen-binding fragment thereof and a pharmaceutical cytotoxin oligomer or a pharmaceutically acceptable salt thereof as described above, wherein the pharmaceutical cytotoxin oligomer is covalently attached to the antibody or the antigen-binding fragment thereof.

In certain embodiments, the antibody-drug conjugate has a general formula (I)

Ab-[L-(T)ₘ]ₙ (I),

wherein Ab is the antibody or antigen-binding fragment thereof; T is the pharmaceutical cytotoxin oligomer; L is an organic linker; and m and n is, independently of each other, an integer from 1 to 10.

In some embodiments, m is an integer from 1 to 5, preferably from 1 to 3. In some embodiments, m is 1. In some embodiments, m is 2. In some embodiments, m is 3.

In some embodiments, n is an integer from 1 to 8, preferably from 2 to 8, more preferably from 4 to 8.

The product m·n is also referred to as the "drug-to-antibody-ratio" (DAR). In preferred embodiments, the DAR is from 1 to 16, i. e. at least one and at most sixteen cytotoxin oligomers bound per antibody. Preferably, the DAR is from 2 to 12, more preferably from 2 to 10 or 2 to 8, most preferred from 4 to 8.

For the purpose of the present invention, the term "antibody" is used in its broadest sense and encompasses monoclonal antibodies, polyclonal antibodies, dimers, multimers, bispecific antibodies or multispecific antibodies, as well as antibody fragments having an antigen-specific binding site, i. e. an antigen binding site that immuno-specifically binds an antigen of a target cell or a part thereof. Preferably, the antibody is a monoclonal antibody (mAb) or antigen-binding fragment thereof. Examples of antibody fragments include Fab, Fab', F(ab')₂, and scFv fragments, single-entity antibodies, also known as a nanobodies, diabodies, linear antibodies, fragments produced by a Fab expression library, anti-idiotypic (anti-Id) antibodies, CDR (complementary determining region), and epitope-binding fragments of any of the above which immuno-specifically bind to e. g. any human or animal antigens, cancer cell antigens, senescent cell antigens, viral antigens or microbial antigens, single-chain antibody molecules, multispecific antibodies formed from antibody fragments. Antibodies may be murine, human, humanized, chimeric, or derived from other species. The antibody may be any type of immunoglobulin, e. g. IgG, IgE, IgM, IgD, or IgA, any class, e. g. IgGl, IgG2, IgG3, IgG4, IgA1 or IgA2, or subclass of immunoglobulin.

In preferred embodiments, the antibody or antigen-binding fragment thereof is specific for a tumor-associated antigen (TAA), i. e. an anti-TAA antibody, or a tumor-specific antigen (TSA), i. e. an anti-TSA antibody. Tumor-specific antigens are found on cancer cells only, not on healthy cells. Tumor-associated antigens have elevated levels on tumor cells, but are also expressed at lower levels on healthy cells. Such tumor-associated antigens and tumor-specific antigens are known in the art. Examples of TAAs and TSAs include, but are not limited to, BMPR1B (bone morphogenetic protein receptor-type IB, Genbank accession no. NM-001203), E16 (LAT1, SLC7A5, Genbank accession no. NM-003486), STEAP1 (six transmembrane epithelial antigen of prostate, Genbank accession no. NM-012449), 0772P (CA125, MUC16, Genbank accession no. AF361486), MPF (MPF, MSLN, SMR, megakaryocyte potentiating factor, mesothelin, Genbank accession no. NM-005823), Napi3b (NAPI-3B, NPTIIb, SLC34A2, solute carrier family 34 (sodium phosphate), member 2, type II sodium-dependent phosphate transporter 3b, Genbank accession no. NM-006424), Sema 5b (F1110372, KIAA1445, Mm.42015, SEMA5B, SEMAG, Semaphorin 5b Hlog, sema entity, seven thrombospondin repeats (type 1 and type 1-like), transmembrane entity (TM) and short cytoplasmic entity, (semaphorin) 5B, Genbank accession no. AB040878), PSCA hlg (2700050C12Rik, C530008016Rik, RIKEN cDNA 2700050C12, RIKEN cDNA 2700050C12 gene, Genbank accession no. AY358628), ETBR (Endothelin type B receptor, Genbank accession no. AY275463), MSG783 (RNF124, hypothetical protein F1120315, Genbank accession no. NM-017763), STEAP2 (HGNC-8639, IPCA-1, PCANAP1, STAMP1, STEAP2, STMP, prostate cancer associated gene 1, six transmembrane epithelial antigen of prostate 2, six transmembrane prostate protein, Genbank accession no. AF455138), TrpM4 (BR22450, F1120041, TRPM4, TRPM4B, transient receptor potential cation channel, subfamily M, member 4, Genbank accession no. NM-017636), CRIPTO (CR, CR1, CRGF, CRIPTO, TDGF1, teratocarcinoma-derived growth factor, Genbank accession no. NP-003203 or NM-003212), CD21 (CR2 (Complement receptor 2) or C3DR(C3d/Epstein Barr virus receptor) or Hs.73792, Genbank accession no. M26004), CD79b (CD79B, CD79β, IGb (immunoglobulin-associated beta), B29, Genbank accession no. NM-000626 or 11038674), FcRH2 (IFGP4, IRTA4, SPAP1A (SH2 entity containing phosphatase anchor protein 1a), SPAP1B, SPAP1C, Genbank accession no. NM-030764, AY358130), HER2 (ErbB2, Genbank accession no. M11730), NCA (CEACAM6, Genbank accession no. M18728), MDP (DPEP1, Genbank accession no. BC017023), IL20Rα (IL20Ra, ZCYTOR7, Genbank accession no. AF184971), Brevican (BCAN, BEHAB, Genbank accession no. AF229053), EphB2R (DRT, ERK, HekS, EPHT3, Tyro5, Genbank accession no. NM-004442), ASLG659 (B7h, Genbank accession no. AX092328), PSCA (Prostate stem cell antigen precursor, Genbank accession no. AJ297436), GEDA (Genbank accession No. AY260763), AAP14954 lipoma HMGIC fusion-partner-like protein/pid=AAP14954.1 Homo sapiens (human), BAFF-R (B cell-activating factor receptor, BLyS receptor 3, BR3, Genbank accession No. AF116456), BAFF receptor/pid=NP-443177.1-Homo sapiens, CD22 (B-cell receptor CD22-B isoform, BL-CAM, Lyb-8, Lyb8, SIGLEC-2, FLJ22814, Genbank accession No. AK026467), CD79a (CD79A, CD79α, immunoglobulin-associated alpha), CXCR5 (Burkitt's lymphoma receptor 1), HLA-DOB (Beta subunit of MHC class II molecule), P2X5 (Purinergic receptor P2X ligand-gated ion channel 5), CD72 (B-cell differentiation antigen CD72, Lyb-2, Genbank accession No. NP-001773.1), LY64 (Lymphocyte antigen 64 (RP105)), FcRH1 (Fc receptor-like protein 1), IRTA2 (FcRH5, Immunoglobulin superfamily receptor translocation associated 2), TENB2 (TMEFF2, tomoregulin, TPEF, HPP1, TR), MUC1 (Tumor-associated MUC1 glycopeptide epitopes).

In some embodiments, the antibody or antigen-binding fragment thereof is specific for a senescent surface marker, i. e. a molecule or protein specifically present on the surface of senescent cells.

In accordance with the ordinary understanding in the field of ADCs, a "linker" is a bifunctional or multifunctional organic molecule which allows to covalently conjugate ("link") the drug moiety or "payload", e. g. the cytotoxin oligomer of the present invention, with an antibody or antigen-binding fragment thereof to form an antibody-drug conjugate. Accordingly, linkers can be defined as the interface between targeting moiety, i. e. the antibody or antigen-binding fragment thereof, and the drug moiety, i. e. the cytotoxin oligomer. Linkers comprise a separate coupling functionality each for binding to the antibody or antigen-binding fragment thereof and for binding to the drug moiety, respectively. Therefore, linkers can be used to further adapt and/or tailor ADCs in terms of efficient ADC construction as well as therapeutic efficiency, mechanism of action, and therapeutic window, e. g. to tackle the overall hydrophobicity of the drug-linker and overcome the limiting tie of a restrained drug loading. Innovative hydrophilicity sources have been explored in various linker frameworks and with different payloads, resulting in ameliorated physicochemical properties, favorable pharmacokinetic profiles, major tolerability, evasion of MDR-related resistance, and efficacy in heterogeneous tumors. One possibility to increase the hydrophilicity of a drug-linker is for instance the introduction of PEG (polyethylene glycol) and PEG units, respectively. Moreover, linkers can be used to control when a payload gets released from the antibody carrier.

There are two genera of linkers, namely so-called "cleavable linkers" and "non-cleavable linkers".

Non-cleavable linkers are stably linked to the drug moiety on one end and the antibody on another end. The drug moiety is therefore only released from the antibody upon receptor-mediated endocytosis and lysosomal processing of the ADC. Non-cleavable linkers are independent of (bio-)chemical signals, also referred to as "triggers". Once the antibody is degraded by lysosomal enzymes, the linker-payload conjugate is released into the intracellular environment and free to exert its cytotoxic activity. When targeting hematological tumors, for example, ADCs based on non-cleavable linkers can be more useful to impart greater specificity and lower toxicity.

The applied art provides the skilled artisan with a wealth of suitable non-cleavable linkers for practicing the present invention. Suitable examples include, but are not limited to, non-cleavable linkers based on maleimidocaproyl (mc) and 4-maleimidomethyl cyclohexane-1-carboxylate (mcc) and non-cleavable linkers containing PEG with alkynes and piperazine.

Cleavable linkers are cleaved in response to specific biochemical or chemical signals ("triggers"). For example, cleavable linkers are cleaved in response to a change in redox potential or pH-value or in presence of enzymes such as hydrolases or oxidoreductases. Cleavable linkers can be used to control drug release from the ADC.

Cleavable linkers can be classified into two subclasses, namely "chemically cleavable linkers" and "enzyme-cleavable linkers".

Chemically cleavable linkers can be further classified as acid cleavable or reducible linkers. Both are clinically established with several FDA and EMA-approved ADCs based on acid cleavable linkers and reducible linkers.

Acid cleavable linkers exploit the natural acidity of endosomes and lysosomes having a pH-value in a range from pH 4.5-6.2, which contrasts with the neutral pH 7.4 of the plasma. Examples of approved ADCs with acid cleavable linkers are Mylotarg and Besponsa. The most commonly used acid-cleavable linkers are hydrazones which are reliably stable at neutral pH and labile at pH 4.5. Other acid labile groups include a combination of carbonate linkers with alcohol-containing payloads.

Reducible or disulfide linkers are the most prominent class of chemically cleavable linkers. This linker chemistry is stable at physiological pH but susceptible to nucleophilic attacks from thiols. In the plasma, only human serum albumin (HSA) contains thiols, but their reactivity is limited due to the reduced solvent exposure of these groups. In contrast, the cytosol is enriched with glutathione (GSH), with exposed and highly reactive thiol groups. Its presence offers the opportunity to use disulfide linkers for highly selective intracellular release of the payload from the ADC. Moreover, elevated GSH levels are often a feature of oxidative stress associated with tumors, which can impart additional selectivity to this type of linker chemistry.

Enzyme-cleavable linkers use enzymes for payload release. An enzyme exploited in the applied art in payload release is e. g. cathepsin B, a cysteine protease cleaving the peptide bond in dipeptide substrates containing valine (Val) and citrulline (Cit) and p-aminobenzylalcohol (PAB). Valine-citrulline is the most commonly used dipeptide linker substrate in currently applied enzyme-dependent linker chemistry. Glycosidase-cleavable linkers are another example of enzyme-cleavable linkers commonly exploited in ADCs. Glucosidases are hydrolytic enzymes that are typically confined to the lysosomal compartment, but like cathepsin B, they can be secreted by tumor cells in necrotic tissues. Non-limiting examples are β-galactosidase cleavable linkers and the more commonly used β-glucuronidase cleavable linkers which are based on hydrolysis of β-D-glucuronic acid residues at lysosomal pH.

Accordingly, a non-cleavable linker is essentially stable outside a cell, i. e. extracellularly. A cleavable linker includes a pre-defined cleavage site which is cleavable by enzymatic activity, hydrolysis, or other metabolic conditions or triggers.

A linker may also include a spacer structure that spatially separates the cytotoxin oligomer from the antibody. The use of linkers is well-known in the art and a skilled artisan is readily capable to select a suitable linker based on his knowledge and the present disclosure and teaching.

In certain embodiments, the linker comprises a coupling functionality that is configured for covalent coupling to a cysteine thiol, an amine, e. g. the N-terminus or an amino acid side-chain such as lysine, or any other modification of the antibody. The linker may be substituted with a sulfonate substituent or other substituents which may increase water solubility of the linker and facilitate the coupling reaction of the linker with the antibody. The coupling functionality may be configured for covalent coupling to a nucleophilic group on an antibody, including, but not limited to, N-terminal amino groups, side-chain amino groups, e. g. of lysine, side-chain thiol groups, e. g. of cysteine, and hydroxyl groups or amino groups of a carbohydrate in case of a glycosylated antibody. Amino groups, thiol groups and hydroxyl groups are nucleophilic and capable of reacting to form covalent bonds with e. g. an electrophilic group of the linker, including, but not limited to, active esters such as N-hydroxysuccinimide (NHS) esters, hydroxybenzotriazole (HOBt) esters, haloformates, and acid halides, alkyl and benzyl halides such as haloacetamides, aldehydes, ketones, carboxyl and maleimide groups. Certain antibodies have reducible interchain disulfides, i. e. cysteine bridges. Antibodies may be made reactive for conjugation with the linker by treatment with a reducing agent such as DTT (dithiothreitol). Additional nucleophilic groups can be introduced into antibodies through the reaction of lysines with 2-iminothiolane (Traut's reagent) resulting in conversion of an amine into a thiol. Reactive thiol groups may be introduced into the antibody by introducing one, two, three, four, or more cysteine residues e. g. by preparing mutant antibodies comprising one or more non-native cysteine residues or through commercially available chemical kits.

In certain embodiments, the linker is a cleavable linker. In preferred embodiments, the linker is less resistant to cleavage under physiological conditions than the crosslinking entity of the pharmaceutical cytotoxin oligomer.

In certain embodiments, the linker is configured to release the pharmaceutical cytotoxin oligomer from the antibody or antigen-binding fragment thereof in an intracellular environment, in particular upon receptor-mediated endocytosis and/or lysosomal processing of the antibody-drug conjugate. In some embodiments, the linker comprises a cleavage site configured for cleavage in response to a chemical or biochemical trigger selected from the group consisting of redox potential, enzyme and pH value. Specifically, the linker can comprise a substrate for intracellular enzymatic cleavage, e. g. a hydrolase and/or oxidoreductase substrate or cleavage site, or a substrate for chemically triggered cleavage, e. g. in response to change of pH or change of reduction potential.

In some embodiments, the linker is a non-cleavable linker. In this way, it can be even more ensured that the payload of the ADC is not prematurely released extracellularly, but only after intracellular uptake and lysosomal degradation of the antibody, thus further improving the pharmaceutical properties and in particular the therapeutic window of the ADC of the present invention.

In another aspect, the invention provides a method for manufacturing a pharmaceutical cytotoxin oligomer as described above.

The method comprises the following steps: A) providing a first organic cytotoxin entity and at least a second cytotoxin entity, wherein each of the first organic cytotoxin entity and the second organic cytotoxin entity is i) an enzyme inhibitor and/or ii) cytotoxic for a target cell after uptake by a transporter protein of the target cell, B) forming a covalent linkage between the first organic cytotoxin entity and the second organic cytotoxin entity that is substantially resistant to cleavage under physiological conditions, thereby forming the cytotoxin oligomer.

In some embodiments, the first organic cytotoxin entity comprises a first coupling functionality and the second organic cytotoxin monomer comprises a second coupling functionality, wherein the second coupling functionality has a complementary chemical reactivity to the first coupling functionality allowing the first coupling functionality and the second coupling functionality to form a covalent bond with each other. In addition, step B) can comprise combining the first organic cytotoxin entity and the second organic cytotoxin entity under conditions which cause the first coupling functionality and the second coupling functionality to form a covalent bond with each other.

In some embodiments, step A) further comprises providing an organic bridging moiety, and step B) comprises forming the covalent linkage between the first organic cytotoxin entity and the second organic cytotoxin entity via the bridging moiety. For example, forming the covalent linkage can comprise forming a first part of the covalent linkage by covalently conjugating the bridging moiety to the first organic cytotoxin entity, and forming a second part of the covalent linkage by covalently conjugating the bridging moiety to the second organic cytotoxin entity, thereby crosslinking the first organic cytotoxin entity and the second organic cytotoxin entity with each other via the bridging moiety.

Accordingly, the bridging moiety is at least a bifunctional molecule, i. e. comprising at least two coupling functionalities, also referred to as "functional groups", configured for, i. e. capable of, forming a covalent bond with the first organic cytotoxin entity and the second organic cytotoxin entity. Naturally, the bridging moiety can also be a multifunctional molecule having a plurality of coupling functionalities configured for forming a covalent bond with additional entities, such as an additional third or further organic cytotoxin entity or a linker, for example. In some embodiments, the bridging moiety is a bifunctional molecule having two coupling functionalities configured for forming a covalent bond with the first organic cytotoxin entity and the second organic cytotoxin entity. In preferred embodiments, the bridging moiety is a trifunctional molecule having three coupling functionalities configured for forming a covalent bond with the first organic cytotoxin entity, the second organic cytotoxin entity and either a third organic cytotoxin entity or a linker, for example. In other preferred embodiments, the bridging moiety is a tetrafunctional molecule having three coupling functionalities configured for forming a covalent bond with the first organic cytotoxin entity, the second organic cytotoxin entity, a third organic cytotoxin entity and either a fourth organic cytotoxin entity or a linker, for example.

In some embodiments, the first organic cytotoxin entity comprises a first coupling functionality, the second organic cytotoxin entity comprises a second coupling functionality and the organic bridging moiety comprises at least a first complementary coupling functionality and a second complementary coupling functionality, wherein the first complementary coupling functionality has a complementary chemical reactivity to the first coupling functionality allowing the first coupling functionality and the first complementary coupling functionality to form a covalent bond with each other, and the second complementary coupling functionality has a complementary chemical reactivity to the second coupling functionality allowing the second coupling functionality and the second complementary coupling functionality to form a covalent bond with each other. In addition, step B) can comprise combining the first organic cytotoxin entity, the second organic cytotoxin entity and the organic bridging moiety under conditions which cause the first coupling functionality and the first complementary coupling functionality to form a covalent bond with each other, and which, preferably at the same time, cause the second coupling functionality and the second complementary coupling functionality to form a covalent bond with each other.

In some embodiments, the first coupling functionality is the same functional group as the second coupling functionality and, accordingly, the first complementary coupling functionality is the same functional group as the second complementary coupling functionality. In other embodiments, the first coupling functionality and the second coupling functionality are different from each other, and, accordingly, the first complementary coupling functionality and the second complementary coupling functionality are different from each other.

In embodiments wherein B) comprises forming the covalent linkage between the first organic cytotoxin entity and the second organic cytotoxin entity via the bridging moiety, it is preferred that the first coupling functionality and the second coupling functionality are either the same or chemically non-complementary to each other, also referred to as having an orthogonal chemical reactivity, thereby barring the first organic cytotoxin entity and the second organic cytotoxin entity to directly form a covalent bond with each other. Likewise, in such embodiments, the first complementary coupling functionality and the second complementary coupling functionality are either the same or chemically non-complementary to each other, thereby barring bridging moieties to form a covalent bond with each other.

As already noted above, the bridging moiety can also comprise a third or further complementary coupling functionality, being the same as the first coupling functionality and the second coupling functionality or different, and having a complementary chemical reactivity to a third or further coupling functionality of a third or further organic cytotoxin entity and/or of a linker. Further possible variations are readily apparent to the skilled artisan on the basis of the disclosure and teaching above.

In some embodiments, the bridging moiety comprises at least one orthogonal coupling functionality having a non-complementary chemical reactivity to the coupling functionalities of the first and second organic cytotoxin entity, e. g. the first coupling functionality and the second coupling functionality, thereby barring the first organic cytotoxin entity and the second organic cytotoxin entity from forming a covalent bond with the bridging moiety through the orthogonal coupling functionality. In this way, the orthogonal coupling functionality remains available on the bridging moiety after forming the cytotoxin oligomer in step B), and can thus be used for coupling the cytotoxin oligomer to another molecule such as a linker and/or antibody, for example. As used herein, the orthogonal coupling functionality may also be a functional group that is protected with a releasable protecting group as already described above.

Preferably, the first coupling functionality and second coupling functionality or, as the case may be, the first coupling functionality and first complementary coupling functionality and the second coupling functionality and second complementary coupling functionality, and, if present, the third or further coupling functionality and the third or further complementary coupling functionality, are mutual reaction partners in any one of the click chemistry reactions described above, such as an azide or tetrazine as the first coupling functionality and an alkyne or alkene as the second coupling functionality; an azide or tetrazine as the first coupling functionality and second coupling functionality and an alkyne or alkene as the first complementary coupling functionality and second complementary coupling functionality, or vice versa, for example. However, the first coupling functionality and second coupling functionality, or, as the case may be, the first coupling functionality and first complementary coupling functionality and the second coupling functionality and second complementary coupling functionality, and, if present, the third or further coupling functionality and the third or further complementary coupling functionality, can of course also be selected from any of the other mutually reactive functional groups described above, such as amino group and carboxylic group, aldehyde group or keto group, for example. The orthogonal coupling functionality is preferably selected from the group consisting of amino group, a carboxyl group, a hydroxyl group, hydroxylamine group, isothiocyanate group, an aldehyde group, a keto group and thiol group, which is optionally protected with a releasable protecting group.

The coupling functionality of each of the organic cytotoxin entities, e. g. the first coupling functionality and the second coupling functionality, can be a functional group of an amino acid naturally occurring in the corresponding first and/or second organic cytotoxin entity such as an amino group, a carboxyl group, a hydroxy group, an aldehyde group, a keto group or a thiol group. In preferred embodiments, however, the coupling functionality of the first organic cytotoxin entity is a functional group that does not occur in a natural counterpart of the first organic cytotoxin entity and/or the coupling functionality of the second organic cytotoxin entity is a functional group that does not occur in a natural counterpart of the second organic cytotoxin entity. In embodiments wherein the first and/or second organic cytotoxin entity is a microcystin or a nodularin, the coupling functionality or functional group, respectively, is preferably biosynthetically or semi-synthetically introduced into the cytotoxin entity as described in WO 2018/219619 A1, for example, the disclosures of which is hereby incorporated by reference in its entirety to more fully describe the technical background to which the present invention pertains. Preferably, the coupling functionality of the first organic cytotoxin entity and/or second organic cytotoxin entity is configured for bioorthogonal conjugation, i. e. a chemical conjugation reaction that can occur inside of living systems without interfering with native biochemical processes. In some embodiments, the coupling functionality of the first organic cytotoxin entity and/or second organic cytotoxin entity is selected from a group consisting of azido group, alkyne group, alkene group, phosphine, phosphonate, tetrazine, hydrazine, hydroxylamine, isothiocyanate, and any combinations thereof.

In some embodiments, the first organic cytotoxin entity and/or the second organic cytotoxin entity has at least 90% or 95% sequence identity with a sequence listed in Tab. 1 below, i. e. the first organic cytotoxin entity and/or the second organic cytotoxin entity has at least 90% or 95% sequence identity with a sequence selected from the group consisting of SEQ ID NO:2 to SEQ ID NO:15, NOD and [D-PrgMeAsp]1-NOD.

In yet another aspect, the invention provides a method for manufacturing an antibody-drug conjugate.

The method comprises the following steps: a) providing a pharmaceutical cytotoxin oligomer or a pharmaceutically acceptable salt thereof as defined above, b) providing an antibody or an antigen-binding fragment thereof, and c) covalently attaching the pharmaceutical cytotoxin oligomer to the antibody or the antigen-binding fragment thereof.

In certain embodiments, method step a) comprises performing the method for manufacturing a pharmaceutical cytotoxin oligomer as defined above.

In certain embodiments, method step c) comprises the following substeps: c1) providing a linker, c2) covalently attaching the pharmaceutical cytotoxin oligomer to the linker, and c3) covalently attaching the linker to the antibody or the antigen-binding fragment thereof. It is understood that the sequence of the substeps is not necessarily predetermined by the numbering.

For example, it is possible to perform substep c2) before or after substep c3). Preferably, however, substep c2) is performed prior to substep c3), i. e. the pharmaceutical cytotoxin oligomer is first covalently attached to the linker, and subsequently the cytotoxin oligomer-linker conjugate is covalently attached via the linker to the antibody.

Suitable laboratory protocols for covalent conjugation of the pharmaceutical cytotoxin oligomer to the antibody or the linker, respectively, and for the covalent conjugation of the linker to the antibody are well-established in the art and readily available for the skilled artisan. In this regard, reference is also made to description above and the detailed description of embodiments below.

In some embodiments, the linker and/or the covalent attachment of the pharmaceutical cytotoxin oligomer to the linker is configured to release the pharmaceutical cytotoxin oligomer from the antibody or antigen-binding fragment thereof upon receptor-mediated endocytosis and/or lysosomal processing of the antibody-drug conjugate. In particular, the linker can comprise a cleavage site configured for cleavage in response to a chemical or biochemical trigger as already described above.

In certain embodiments, at least one of the first organic cytotoxin entity, the second organic cytotoxin entity and the crosslinking entity of the pharmaceutical cytotoxin oligomer comprises a first coupling functionality and the linker comprises a second coupling functionality having a complementary chemical reactivity to the first coupling functionality for forming a covalent bond with each other. In addition, substep c2) can comprise combining the pharmaceutical cytotoxin oligomer and the linker under conditions which cause the first coupling functionality and the second coupling functionality to form a covalent bond with each other.

In certain embodiments, the invention provides a pharmaceutical cytotoxin oligomer or a pharmaceutically acceptable salt thereof as defined above or an antibody-drug conjugate as defined above for use as a medicament, more particularly for use in the treatment of a malignant disease or age-associated morbidities.

In certain embodiments, the invention provides a method for treating a malignant disease in a subject, in particular a human subject, comprising administering to the subject a pharmaceutically effective amount or a therapeutically effective dosing regimen of a pharmaceutical cytotoxin oligomer or a pharmaceutically acceptable salt thereof as defined above or a pharmaceutically effective amount of an antibody-drug conjugate as defined above.

In certain embodiments, the malignant disease is cancer. Examples of cancer to be treated herein include, but are not limited to, carcinoma, lymphoma, blastoma, sarcoma, and leukemia or lymphoid malignancies. More particular examples of such cancers include squamous cell cancer, e. g. epithelial squamous cell cancer, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, gastrointestinal stromal tumor (GIST), pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. The cancer can be characterized by overexpression of HER2 or ErbB receptor, for example.

A pharmaceutically effective amount can, for example, be 0.01-100 mg per kg of bodyweight (mg/kg), more particularly 0.1-20 mg/kg or 1-10 mg/kg. A therapeutically effective dosing regimen can, for example, comprise administering a single dose or a plurality of doses of 0.01-100 mg/kg, more particularly 0.1-20 mg/kg or 1-10 mg/kg.

It is understood that the various embodiments described for one aspect of the invention are also applicable to the other aspects of the invention. Accordingly, features and functions disclosed above and in the following in connection with the cytotoxin oligomer may therefore also relate to the antibody-drug conjugate as well as the corresponding uses and methods of manufacture thereof, and vice versa.

### Brief description of figures

- Fig. 1: structural formula of a microcystin homodimer according to the present invention;
- Fig. 2: structural formula of a further microcystin homodimer according to the present invention;
- Fig. 3: structural formula of a microcystin heterodimer according to the present invention;
- Fig. 4: structural formula of a microcystin homotrimer according to the present invention;
- Fig. 5: scheme of semi-synthetic production of [D-EdaMeAsp]¹-NOD (A) and [D-PrgMeAsp]¹-NOD (B) monomers;
- Fig. 6: HPLC chromatogram of product mixture obtained from semi-synthetic production of [D-PrgMeAsp]¹-NOD monomers;
- Fig. 7: structural formula of a nodularin homodimer according to the present invention;
- Fig. 8: structural formula of a nodularin homotrimer according to the present invention;
- Fig. 9: structural formula of an amatoxin homodimer according to the present invention;
- Fig. 10: structural formula of a amatoxin homotrimer according to the present invention;
- Fig. 11: structural formula of the microcystin homodimer of Fig. 2 conjugated with a cleavable linker maleimidocaproyl-Val-Ala-(p-amino-benzyl) carbamate configured for antibody conjugation;
- Fig. 12: column diagrams showing the results from assessment of general cytotoxicity on OATP1B1 and OATP1B3 expressing cell lines (A) and protein phosphatase inhibition (B) for the microcystin homodimers of Fig. 1 and Fig. 2 and, as references, the counterpart monomer and natural microcystin MC-LR.

### Detailed description of embodiments

The present invention is explained in more detail below on the basis of exemplary embodiments with reference to the enclosed figures. Neither the examples nor the figures shall be considered limiting.

### Example 1: Synthesis of monomeric organic cytotoxin entities

The following examples include several microcystins, nodularins and amatoxins as representative species of the genus of organic cytotoxin entities which exert their cytotoxic activity through inhibition of intracellular enzymes of a target cell after being actively transported into the target cell by a transporter protein of the target cell (Tab. 1).

Unless otherwise described in the following examples, the microcystin (MC) monomers were obtained by precursor-directed biosynthesis as described in WO 2018/219619 A1. Briefly, a cyanobacterial strain producing natural microcystins, such as PCC 7820 (Pasteur Culture Collection), was cultivated under conditions allowing for growth of the strain. During the cultivation, the inorganic cultivation medium was supplemented with one of the amino acids propargyltyrosine (PrgTyr), propargyllysine (PrgLys), azidophenylalanine (AzPhe), azidolysine (AzLys), azidonorvaline (AzNva), *N_{ε}-tert-*butyloxycarbonyl-ornithine (Orn(Boc)) or D-azidoalanine (D-AzAla), for example. Each of these amino acids contains a coupling functionality configured for covalent crosslinking with another coupling functionality with complementary chemical reactivity. The supplemented amino acids have been incorporated into the microcystins during the cultivation of the strain, thereby resulting in the biosynthesis of a non-naturally occurring microcystin derivative with a functional group configured for covalent conjugation (the "coupling functionality") in position Aa¹, X², Z⁴ or Aa⁷ (the "coupling site"). Incorporation of the coupling functionality in position Aa³ is generally also possible. After the cultivation, the biomass was harvested and extracted and the microcystin derivatives were purified by means of HPLC. The purified microcystin derivatives served as organic cytotoxin entities for production of cytotoxin oligomers according to the present invention. In case of Orn(Boc) containing microcystin derivatives, the Boc-group is cleaved in trifluoroacetic acid (TFA)/H20/acetonitrile (can) (1:1:1, v/v/v) and stirred for 90 min to expose the free amino function, followed by evaporation and isolation by RP-HPLC. Suitable microcystins can also be synthesised by total chemical synthesis (e. g. Zemskov et al., J Org Chem 2017, 82, 3680-91).

Moreover, semi-synthetically modified variants of microcystins, nodularins and amatoxins can be produced from naturally occurring cytotoxin monomers by chemical incorporation of a coupling functionality configured for covalent crosslinking with another coupling functionality with complementary chemical reactivity.

For instance, semi-synthetically modified variants of the naturally occurring microcystin monomer MC-LR were obtained by Michael addition of amines, thiols or other Michael donors to the Mdha residue (Michael acceptor) in position Aa⁷. Thus, additional coupling functionalities (e. g. an amine, alkyne or azide) were chemically incorporated allowing the selective reaction with complementary functionality as described in example 11 below.

Additionally, semi-synthetically modified variants of a native microcystin monomer can be obtained by chemical modification of the carboxyl group in position Aa³ in accordance with the modifications described below for the carboxyl group in position Aa¹ of nodularin monomers.

The nodularin (NOD) monomer included in the examples is a naturally occurring nodularin monomer obtained via cultivation of a nodularin-producing cyanobacterial strain such as PCC 73104 as described elsewhere, and subsequent extraction and isolation of the nodularin from the biomass by means of HPLC, for example. Suitable nodularin monomers can also be obtained by precursor-directed biosynthesis as described in WO 2018/219619 A1.

As described above for microcystins, also nodularins can be semi-synthetically modified by Michael addition with Michael donors and the Mdhb/Dhb group (Michael acceptor) in position Aa⁵ to incorporate additional coupling functionalities.

In addition, semi-synthetically modified variants of the nodularin monomer with an amino or alkyne coupling functionality in position Aa¹ are obtained by amide bond formation between the carboxyl group in position Aa¹ and an amino group as described in example 34 and 35 below.

The amatoxin (AMA) monomers included in the examples are naturally occurring α-amanitin and its derivative amaninamide which lacks the 6-OH on Trp². The amatoxins are obtained either from wild carpophores (fruiting body) of *Amanita* species (e. g. *Amanita phalloides)* collected from natural habitats or from cultured mycelia of *Amanita* species (e.g. *Amanita exitialis)* as described by Zhang et al. (FEMS Microbiol Lett 2005, 252(2), 223-08) and subsequent extraction and isolation of the amatoxin from the biomass by HPLC, for example. The α-amanitin monomers have hydroxyl groups as coupling functionalities in position Aa¹ (Dhil = (2S,3R,4R)-4,5-dihydroxy-isoleucine), in position Aa² (Htp = 6-Hydroxytryptophan) and in position Aa⁸ (Hyp = *trans-4-*hydroxyproline). However, the Hyp residue has a major impact on the bioactivity and the corresponding cytotoxicity of the amatoxins, therefore it is not considered as suitable ligation site (Matinkhoo et al., Chem. Eur. J. 2021, 27, 10282-92). Suitable amatoxins can also be synthesised by total chemical synthesis (e. g Siegert et al., Angew Chem 2020, 59, 5500-04; Lutz et al., Angew Chem 2020, 59, 11390-93).

Semi-synthetically modified variants of the α-amanitin monomers with an amino coupling group in position Aa² and an alkyne coupling group in position Aa¹ are obtained as described in examples 42 and 43 below.

**Tab. 1: Microcystins (MC), nodularins (NOD) and amatoxins (AMA) used in the examples as monomeric cytotoxin entities. PrgTyr = propargyltyrosine, PrgLys = propargyllysine, AzPhe = azidophenylalanine, AzLys = azidolysine, AzNva = azidonorvaline, Orn = ornithine, D-AzAla = D-azidoalanine, AzProMDap = 3-N-azidopropyl-2-N-Methyl-2,3-diaminopropionic acid, D-EdaMeAsp = D-β-ethylen-1,2-diamine-β-methylaspartic acid, D-PrgMeAsp = D-erythro-β-propargylamine-β-methylaspartic acid, AeHtp = 6-(2-aminoethoxy)tryptophan, PrgDhil = 4,5-[(oxycarbonyl)-propargylamine]-dihydroxyisoleucine.**

| **Name** | **Coupling functionality** | **Coupling position** | **Sequence** | **SEQ ID NO** |
|---|---|---|---|---|
| MC-PrgTyr-Arg | Alkyne | X² | cyclo(D-Ala-PrgTyr-D-MeAsp-Arg-Adda-D-Glu-Mdha) | 2 |
| MC-PrgLys-Arg | Alkyne | X² | cyclo(D-Ala-PrgLys-D-MeAsp-Arg-Adda-D-Glu-Mdha) | 3 |
| MC-AzPhe-Arg | Azide | X² | cyclo(D-Ala-AzPhe-D-MeAsp-Arg-Adda-D-Glu-Mdha) | 4 |
| MC-AzLys-Arg | Azide | X² | cyclo(D-Ala-AzLys-D-MeAsp-Arg-Adda-D-Glu-Mdha) | 5 |
| MC-AzNva-Arg | Azide | X² | cyclo(D-Ala-AzNva-D-MeAsp-Arg-Adda-D-Glu-Mdha) | 6 |
| MC-Leu-PrgTyr | Alkyne | Z⁴ | cyclo(D-Ala-Leu-D-MeAsp-PrgTyr-Adda-D-Glu-Mdha) | 7 |
| MC-Leu-AzLys | Azide | Z⁴ | cyclo(D-Ala-Leu-D-MeAsp-AzLys-Adda-D-Glu-Mdha) | 8 |
| MC-Leu-Orn | Amino | Z⁴ | cyclo(D-Ala-Leu-D-MeAsp-Orn-Adda-D-Glu-Mdha) | 9 |
| MC-Leu-PrgLys | Alkyne | Z⁴ | cyclo(D-Ala-Leu-D-MeAsp-PrgLys-Adda-D-Glu-Mdha) | 10 |
| [D-AzAla]¹MC-WR | Azide | Aa¹ | cyclo(D-AzAla-Trp-D-MeAsp-Arg-Adda-D-Glu-Mdha) | 11 |
| [AzProMDap]⁷MC-LR | Azide | Aa⁷ | cyclo(D-Ala-Leu-D-MeAsp-Arg-Adda-D-Glu-AzProMDap) | 12 |
| MC-Tyr-AzNva | Azide | Z⁴ | cyclo(D-Ala-Tyr-D-MeAsp-AzNva-Adda-D-Glu-Mdha) | 13 |
| [D-EdaMeAsp]¹-NOD | Amino | Aa¹ | cyclo[D-EdaMeAsp-Arg-Adda-D-Glu-Mdhb] | - |
| [D-PrgMeAsp]¹-NOD | Alkyne | Aa¹ | cyclo[D-PrgMeAsp-Arg-Adda-D-Glu-Mdhb] | - |
| [AeHtp]²-AMA | Amino | Aa² | cyclo{Dhil-cyclo[AeHtp-Gly-Cys-Gly-Cys]-Asn-Hyp} | 14 |
| [PrgDhil]¹-AMA | Alkyne | Aa¹ | cyclo{PrgDhil-cyclo[Trp-Gly-Cys-Gly-Cys]-Asn-Hyp} | 15 |

### Example 2: Synthesis of a homomeric microcystin dimer by crosslinking of MC-PrgTyr-Arg monomers with a bifunctional bridging moiety

A homomeric dimer derived from two MC-PrgTyr-Arg monomers (SEQ ID NO:2) described in example 1 as both the first organic cytotoxin entity and the second organic cytotoxin entity was produced. The MC-PrgTyr-Arg monomers have an alkyne group as coupling functionality in position X².

The MC-PrgTyr-Arg monomers were dimerized using the bridging molecule of structural formula BM-I to create the crosslinking entity which forms a covalent linkage between the first organic cytotoxin entity and the second organic cytotoxin entity. For this purpose, the bridging molecule BM-I carries two terminal azido groups as complementary coupling functionalities:

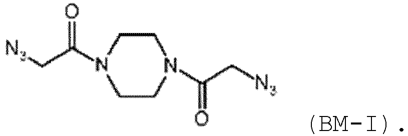

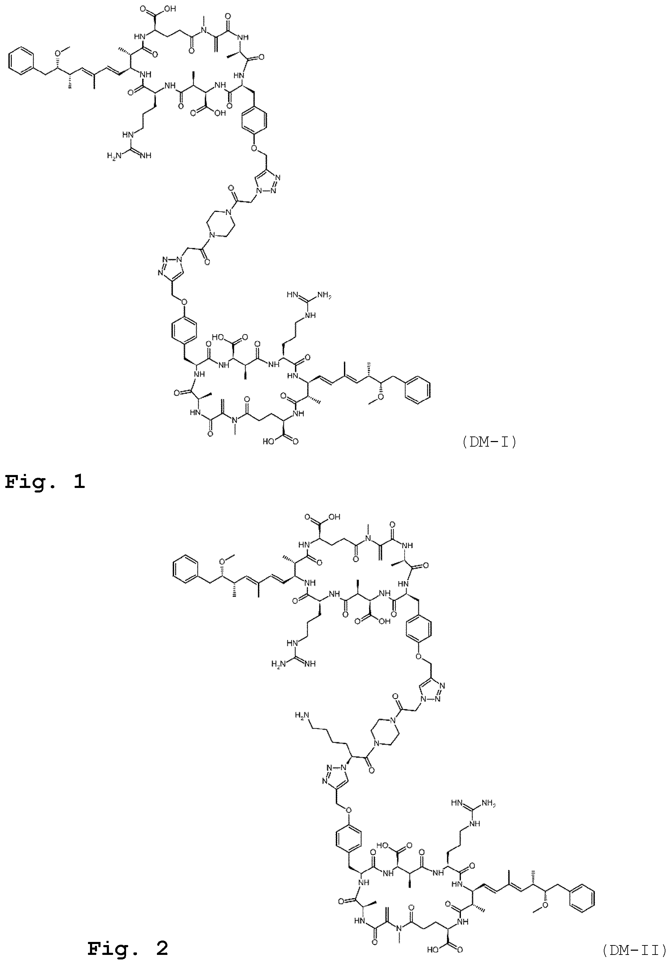

Crosslinking between the alkyne coupling functionalities in position X² of the two MC-PrgTyr-Arg monomers and the two terminal azido groups of BM-I as complementary coupling functionalities was achieved by copper-catalyzed azide-alkyne-cycloaddition (CuAAC). Briefly, CuAAC was performed using a reaction mixture of 2 equivalents (eq.) of the monomer in dimethyl sulfoxide (DMSO), 1 eq. of BM-I, 10 eq. of 100 mM CuSO₄ aqueous solution, and 20 eq. of 200 mM tris((1-hydroxy-propyl-1H-1,2,3-triazol-4-yl)¬methyl)amine (THPTA) aqueous solution. The reaction was started by adding 10 eq. of 100 mM sodium ascorbate aqueous solution. The reaction product was isolated by semi-preparative C-18 RP-HPLC and lyophilized. The structural formula DM-I of the homomeric microcystin dimer is shown in Fig. 1.

### Example 3: Synthesis of a homomeric microcystin dimer by crosslinking of MC-PrgLys-Arg monomers with a bifunctional bridging moiety

A homomeric dimer derived from two MC-PrgLys-Arg monomers (SEQ ID NO:3) described in example 1 as both the first organic cytotoxin entity and the second organic cytotoxin entity is produced. The MC-PrgLys-Arg monomers have an alkyne group as coupling functionality in position X². The MC-PrgLys-Arg monomers are dimerized using the bridging molecule of structural formula BM-I in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 4: Synthesis of a homomeric microcystin dimer by crosslinking of MC-AzPhe-Arg monomers with a bifunctional bridging moiety

A homomeric dimer derived from two MC-AzPhe-Arg monomers (SEQ ID NO:4) described in example 1 as both the first organic cytotoxin entity and the second organic cytotoxin entity is produced. The MC-AzPhe-Arg monomers have an azide group as coupling functionality in position X². The MC-AzPhe-Arg monomers are dimerized using the bridging molecule of structural formula BM-II to create the crosslinking entity which forms a covalent linkage between the first organic cytotoxin entity and the second organic cytotoxin entity. For this purpose, the bridging molecule BM-II carries two terminal alkyne groups as complementary coupling functionalities:

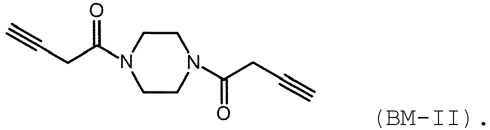

Crosslinking between the azide coupling functionalities in position X² of the two MC-AzPhe-Arg monomers and the two terminal alkyne groups of BM-II as complementary coupling functionalities is accomplished by CuAAC as described in example 2 above. The reaction product is isolated by semi-preparative C-18 RP-HPLC and lyophilized.

### Example 5: Synthesis of a homomeric microcystin dimer by crosslinking of MC-AzLys-Arg monomers with a bifunctional bridging moiety

A homomeric dimer derived from two MC-AzLys-Arg monomers (SEQ ID NO:5) described in example 1 as both the first organic cytotoxin entity and the second organic cytotoxin entity is produced. The MC-AzLys-Arg monomers have an azide group as coupling functionality in position X². The MC-AzLys-Arg monomers are dimerized using the bridging molecule of structural formula BM-II in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 6: Synthesis of a heteromeric microcystin dimer by crosslinking monomers of MC-AzNva-Arg and MC-AzPhe-Arg with a bifunctional bridging moiety

A heteromeric dimer derived from a MC-AzNva-Arg monomer (SEQ ID NO:6) described in example 1 as the first organic cytotoxin entity and a MC-AzPhe-Arg monomer (SEQ ID NO:4) described in example 1 as the second organic cytotoxin entity is produced. Both monomers have an azide group as coupling functionality in position X². The MC-AzNva-Arg monomer (1 eq.) and the MC-AzPhe-Arg (1 eq.) are dimerized using the bridging molecule of structural formula BM-II in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 7: Synthesis of a homomeric microcystin dimer by crosslinking of MC-Leu-PrgTyr monomers with a bifunctional bridging moiety

A homomeric dimer derived from two MC-Leu-PrgTyr monomers (SEQ ID NO:7) described in example 1 as both the first organic cytotoxin entity and the second organic cytotoxin entity is produced. The MC-Leu-PrgTyr monomers have an alkyne group as coupling functionality in position Z⁴. The MC-Leu-PrgTyr monomers are dimerized using the bridging molecule of structural formula BM-I in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 8: Synthesis of a homomeric microcystin dimer by crosslinking of MC-Leu-AzLys monomers with a bifunctional bridging moiety

A homomeric dimer derived from two MC-Leu-AzLys monomers (SEQ ID NO:8) described in example 1 as both the first organic cytotoxin entity and the second organic cytotoxin entity is produced. The MC-Leu-AzLys monomers have an azide group as coupling functionality in position Z⁴. The MC-Leu-AzLys monomers are dimerized using the bridging molecule of structural formula BM-II in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 9: Synthesis of a homomeric microcystin dimer by crosslinking of MC-Leu-Orn monomers with a bifunctional bridging moiety

A homomeric dimer derived from two MC-Leu-Orn monomers (SEQ ID NO:9) described in example 1 as both the first organic cytotoxin entity and the second organic cytotoxin entity is produced. The MC-Leu-Orn monomers have an amino group as coupling functionality in position Z⁴. The MC-Leu-Orn monomers are dimerized using the bridging molecule of structural formula BM-III to create the crosslinking entity which forms a covalent linkage between the first organic cytotoxin entity and the second organic cytotoxin entity. For this purpose, the bridging molecule BM-III carries two terminal carboxyl groups as complementary coupling functionalities:

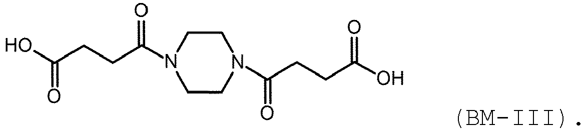

Crosslinking between the amino coupling functionalities in position Z⁴ of the two MC-Leu-Orn monomers and the two terminal carboxyl groups of BM-III as complementary coupling functionalities is accomplished by amide bond formation.

Briefly, the bridging molecule BM-III (1 eq. in DMF) is preincubated with *O*-(7-Azabenzotriazol-1-yl)-*N*,*N*,*N*'*,N'-*tetramethyluronium hexafluorophosphate (HATU) (1.95 eq.) and N,N-Diisopropylethylamine (DIPEA; 4 eq.) for 30 min. Afterwards, the amino group containing monomer (2 eq.) is added to the solution, which is then stirred over night at room temperature. The reaction product is isolated by semi-preparative C-18 RP-HPLC and lyophilized.

### Example 10: Synthesis of a heteromeric microcystin dimer by crosslinking monomers of MC-Leu-PrgLys and MC-Leu-PrgTyr with a bifunctional bridging moiety

A heteromeric dimer derived from a MC-Leu-PrgLys monomer (SEQ ID NO:10) described in example 1 as the first organic cytotoxin entity and a MC-Leu-PrgTyr monomer (SEQ ID NO:7) described in example 1 as the second organic cytotoxin entity is produced. Both monomers have an alkyne group as coupling functionality in position Z⁴. The MC-Leu-PrgLys monomer (1 eq.) and the MC-Leu-PrgTyr monomer (1 eq.) are dimerized using the bridging molecule of structural formula BM-I in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Example 11: Synthesis of a heteromeric microcystin dimer by crosslinking monomers of [D-AzAla]¹MC-WR and [AzproMDap]⁷MC-LR with a bifunctional bridging moiety

A heteromeric dimer derived from a [D-AzAla]¹MC-WR monomer (SEQ ID NO:11) described in example 1 as the first organic cytotoxin entity and a [AzproMDap]⁷MC-LR monomer (SEQ ID NO:12) as the second organic cytotoxin entity is produced.

The monomer [D-AzAla]¹MC-WR monomer is obtained by precursor-directed biosynthesis as described in example 1. The monomer [AzproMDap]⁷MC-LR was semi-synthetically prepared by Aza-Michael-Addition from a natural microcystin MC-LR precursor (SEQ ID NO:16). To this end, MC-LR (1 eq.) was dissolved in DMSO and added to a 1% (w/v) K₂CO₃ aqueous solution which contains 3-azido-1-propanamine (40 eq.). The reaction was stirred overnight at 40 °C. The obtained [AzproMDap]⁷MC-LR was isolated by semi-preparative C-18 RP-HPLC and lyophilized.

The [D-AzAla]¹MC-WR monomer has an azide as coupling functionality in position Aa¹. The [AzproMDap]⁷MC-LR monomer has an azide as coupling functionality in position Aa⁷. The monomers (1 eq. each) are dimerized using the bridging molecule of structural formula BM-II in conjunction with the CuAAC protocol described in example 2 above to create the crosslinking entity which forms a covalent linkage between the first organic cytotoxin entity and the second organic cytotoxin entity. The reaction product is isolated by semi-preparative C-18 RP-HPLC and lyophilized.

### Examples 12-21: Synthesis of homomeric and heteromeric microcystin dimers by crosslinking monomers of examples 2-11 with a trifunctional bridging moiety having an additional antibody-coupling functionality

In examples 12-21, the homomeric dimers and heteromeric dimers of examples 2-11 are re-produced using different bridging molecules to create the crosslinking entity which forms the covalent linkage between the first organic cytotoxin entity and the second organic cytotoxin entity. In examples 12-21, each bridging molecule carries two coupling functionalities with complementary chemical reactivity to the coupling functionalities of the microcystin monomers as described in examples 2-11, and an additional third coupling functionality configured for subsequent linkage of the dimers to an antibody or antigen-binding fragment thereof.

For this purpose, the bridging molecule BM-IV carries two azides as complementary coupling functionalities for crosslinking of microcystin monomers containing alkyne coupling functionalities and a terminal amino group as a third coupling functionality for linkage of the dimer to an antibody or antigen-binding fragment thereof:

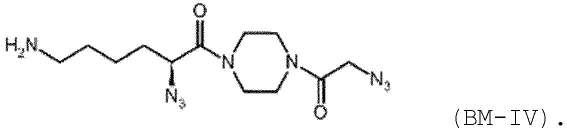

The bridging molecule BM-V carries two alkynes as complementary coupling functionalities for crosslinking of microcystin monomers containing azide coupling functionalities and a terminal amino group as a third coupling functionality for linkage of the dimer to an antibody or antigen-binding fragment thereof:

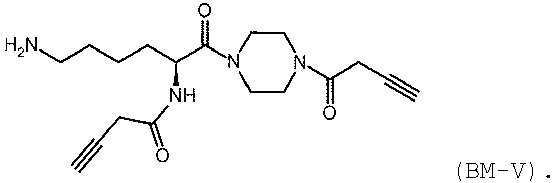

The bridging molecule BM-VI the carries two carboxyl groups as complementary coupling functionalities for crosslinking of microcystin monomers containing amino groups as coupling functionalities and a terminal Boc-protected amino group as a third coupling functionality. After the dimerization reaction, the Boc-group is cleaved in TFA/H₂O/ACN (1:1:1, v/v/v), exposing a free amino group for linkage of the dimer to an antibody or antigen-binding fragment thereof:

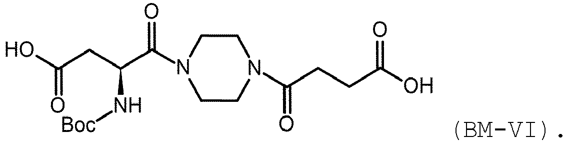

Tab. 2 provides a summary of the dimers produced in examples 12-21.

**Tab. 2: Microcystin dimers produced in examples 12-21. PrgTyr = propargyltyrosine, PrgLys = propargyllysine, AzPhe = azidophenylalanine, AzLys = azidolysine, Aznva = azidonorvaline, Orn = ornithine, D-AzAla = D-azidoalanine, AzProMDap = 3-N-azidopropyl-2-N-Methyl-2,3-diaminopropionic acid.**

| **Example** | **Type** | **Monomer** | **Crosslinking site** | **Bridging molecule** |
|---|---|---|---|---|
| 12 | Homodimer | MC-PrgTyr-Arg | X²-X² | BM-IV |
| 13 | Homodimer | MC-PrgLys-Arg | X²-X² | BM-IV |
| 14 | Homodimer | MC-AzPhe-Arg | X²-X² | BM-V |
| 15 | Homodimer | MC-AzLys-Arg | X²-X² | BM-V |
| 16 | Heterodimer | MC-AzNva-Arg, MC-AzPhe-Arg | X²-X² | BM-V |
| 17 | Homodimer | MC-Leu-PrgTyr | Z⁴-Z⁴ | BM-IV |
| 18 | Homodimer | MC-Leu-AzLys | Z⁴-Z⁴ | BM-V |
| 19 | Homodimer | MC-Leu-Orn | Z⁴-Z⁴ | BM-VI |
| 20 | Heterodimer | MC-Leu-PrgLys, MC-Leu-PrgTyr | Z⁴-Z⁴ | BM-IV |
| 21 | Heterodimer | [D-AzAla]¹MC-WR, [AzproMDap]⁷MC-LR | Aa¹-Aa⁷ | BM-V |

Dimerization using the bridging molecule of structural formula BM-IV or the bridging molecule of structural formula BM-V is achieved with the CuAAC protocol described in example 2, wherein 2 eq. of microcystin monomer are used for homodimers, whereas 1 eq. of each microcystin monomer is used for heterodimers. Dimerization using the bridging molecule of structural formula BM-VI is achieved with the amide bond formation protocol described in example 9.

The reaction products are isolated by semi-preparative C-18 RP-HPLC and lyophilized.

As an illustrative embodiment, Fig. 2 shows the structural formula DM-II of the homomeric microcystin dimer of example 12. CuAAC between the propargyl coupling functionalities of the MC-PrgTyr-Arg monomers and the complementary azide coupling functionalities of the bridging molecule BM-IV forms two triazole rings that covalently crosslink both microcystin entities in position X² via two opposite branches of the bridging moiety. A third branch of the bridging moiety carries a terminal amino group with an orthogonal reactivity to the other coupling functionalities, thereby remaining available after the dimer formation for subsequent linkage of the dimer to an antibody or antigen-binding fragment thereof.

### Example 22: Synthesis of a heteromeric microcystin dimer by direct crosslinking of monomers MC-PrgTyr-Arg and MC-Tyr-AzNva

A heteromeric dimer derived from a MC-PrgTyr-Arg monomer (SEQ ID NO:2) described in example 1 as the first organic cytotoxin entity and a MC-Tyr-AzNva monomer (SEQ ID NO:13) described in example 1 as the second organic cytotoxin entity is produced.

The monomer MC-PrgTyr-Arg has an alkyne coupling functionality in position X², whereas the monomer MC-Tyr-AzNva has an azido group as coupling functionality in position Z⁴, which allows dimerization of both monomers by direct crosslinking using CuAAC. To this end, CuAAC is performed with equimolar amounts of each monomer (1 eq. in DMSO), 100 mM CuSO₄ solution (5 eq.), and 200 mM THPTA solution (10 eq.). The reaction is started by adding 100 mM sodium ascorbate solution (5 eq.). The reaction product is isolated by semi-preparative C-18 RP-HPLC and lyophilized.

### Example 23: Synthesis of a heteromeric microcystin dimer by direct crosslinking of monomers MC-AzLys-Arg and MC-Leu-PrgLys

A heteromeric dimer derived from a MC-AzLys-Arg (SEQ ID NO:5) described in example 1 as the first organic cytotoxin entity and a MC-Leu-PrgLys monomer (SEQ ID NO:10) described in example 1 as the second organic cytotoxin entity is produced.

The monomer MC-AzLys-Arg has an azido group as coupling functionality in position X², whereas the monomer MC-Leu-PrgLys has a propargyl group as coupling functionality in position Z⁴, which allows dimerization of both monomers by direct crosslinking using the CuAAC protocol described in example 22 above. The reaction product is isolated by semi-preparative C-18 RP-HPLC and lyophilized.

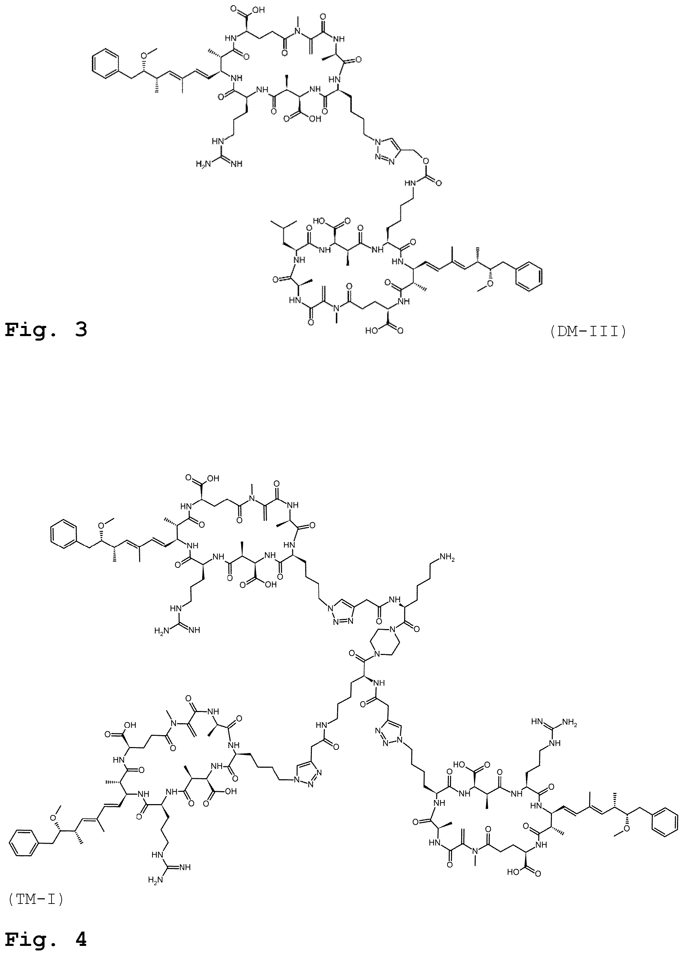

As an illustrative embodiment, Fig. 3 shows the structural formula DM-III of the heteromeric microcystin dimer of example 23. Direct CuAAC between the azido coupling functionality of the MC-AzLys-Arg monomer and the complementary alkyne coupling functionality of the MC-Leu-PrgLys monomer forms a crosslinking entity composed of a triazole ring that covalently crosslink both microcystin entities via amino acid side-chains in positions X² and Z⁴. A carboxyl group in the side-chain of D-MeAsp in position Aa³ is available for linkage of the dimer to an antibody or antigen-binding fragment thereof.

### Example 24: Synthesis of a heteromeric microcystin dimer by direct crosslinking of monomers MC-AzLys-Arg and MC-PrgLys-Arg

A heteromeric dimer derived from a MC-AzLys-Arg monomer (SEQ ID NO:5) described in example 1 as the first organic cytotoxin entity and a MC-PrgLys-Arg monomer (SEQ ID NO:3) described in example 1 as the second organic cytotoxin entity is produced.

The monomer MC-AzLys-Arg has an azido group as coupling functionality in position X², whereas the monomer MC-PrgLys-Arg has a propargyl group as coupling functionality in position X², which allows dimerization of both monomers by direct crosslinking using the CuAAC protocol described in example 22 above. The reaction product is isolated by semi-preparative C-18 RP-HPLC and lyophilized.

### Example 25: Synthesis of a heteromeric microcystin dimer by direct crosslinking of monomers MC-Leu-AzLys and MC-Leu-PrgLys

A heteromeric dimer derived from a MC-Leu-AzLys monomer (SEQ ID NO:8) described in example 1 as the first organic cytotoxin entity and a MC-Leu-PrgLys monomer (SEQ ID NO:10) described in example 1 as the second organic cytotoxin entity is produced.

The monomer MC-Leu-AzLys has an azido group as coupling functionality in position Z⁴, whereas the monomer MC-Leu-PrgLys has a propargyl group as coupling functionality in position Z⁴, which allows dimerization of both monomers by direct crosslinking using the CuAAC protocol described in example 21 above. The reaction product is isolated by semi-preparative C-18 RP-HPLC and lyophilized.

### Example 26: Synthesis of a homomeric microcystin trimer by crosslinking monomers of MC-AzLys-Arg with a trifunctional bridging moiety

A homomeric trimer derived from three MC-AzLys-Arg monomers (SEQ ID NO:5) described in example 1 as the first, second and third organic cytotoxin entity is produced. The MC-AzLys-Arg monomers have an azido group as coupling functionality in position X².

The MC-AzLys-Arg monomers are trimerized using the bridging molecule of structural formula BM-VII to create the crosslinking entity which forms a covalent linkage between the first, second and third organic cytotoxin entity. For this purpose, the bridging molecule BM-VII carries three terminal alkyne groups as complementary coupling functionalities:

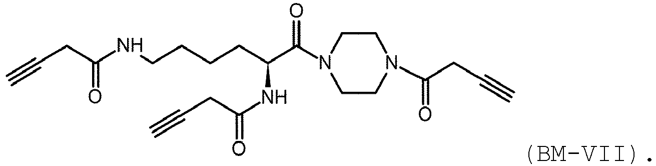

Crosslinking between the azido coupling functionalities in position X² of the three MC-AzLys-Arg monomers and the three terminal alkyne groups of BM-VII as complementary coupling functionalities is achieved by CuAAC. For trimer formation, CuAAC is performed using 3 eq. of the monomer in DMSO, 1 eq. of the bridging molecule, 100 mM CuSO₄ solution (15 eq.), and 200 mM THPTA (30 eq.). The reaction is started by adding 100 mM sodium ascorbate solution (15 eq.). The reaction product is isolated by semi-preparative C-18 RP-HPLC and lyophilized.

### Example 27: Synthesis of a homomeric microcystin trimer by crosslinking monomers of MC-PrgTyr-Arg with a trifunctional bridging moiety

A homomeric trimer derived from three MC-PrgTyr-Arg monomers (SEQ ID NO:2) described in example 1 as the first, second and third organic cytotoxin entity is produced. The MC-PrgTyr-Arg monomers have an alkyne group as coupling functionality in position X².

The MC-PrgTyr-Arg monomers are trimerized using the bridging molecule of structural formula BM-VIII to create the crosslinking entity which forms a covalent linkage between the first, second and third organic cytotoxin entity. For this purpose, the bridging molecule BM-VIII carries three terminal azido groups as complementary coupling functionalities:

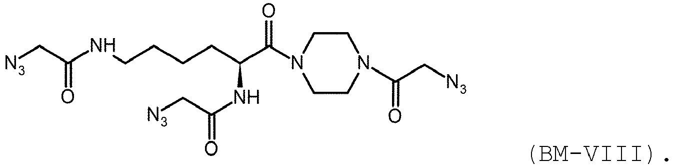

Crosslinking between the alkyne coupling functionalities in position X² of the three MC-PrgTyr-Arg monomers and the three terminal azido groups of BM-VIII as complementary coupling functionalities is achieved by CuAAC according to the protocol described in example 26 above. The reaction product is isolated by semi-preparative C-18 RP-HPLC and lyophilized.

### Example 28: Synthesis of a homomeric microcystin trimer by crosslinking monomers of MC-Leu-AzLys with a trifunctional bridging moiety

A homomeric trimer derived from three MC-Leu-AzLys monomers (SEQ ID NO:8) described in example 1 as the first, second and third organic cytotoxin entity is produced. The MC-Leu-AzLys monomers have an azido group as coupling functionality in position Z⁴.

The MC-Leu-AzLys monomers are trimerized using the bridging molecule of structural formula BM-VII in conjunction with the CuAAC protocol described in example 26 above to create the crosslinking entity which forms a covalent linkage between the first, second and third organic cytotoxin entity. The reaction product is isolated by semi-preparative C-18 RP-HPLC and lyophilized.

### Example 29: Synthesis of a homomeric microcystin trimer by crosslinking monomers of MC-Leu-PrgLys with a trifunctional bridging moiety

A homomeric trimer derived from three MC-Leu-PrgLys monomers (SEQ ID NO:10) described in example 1 as the first, second and third organic cytotoxin entity is produced. The MC-Leu-PrgLys monomers have an alkyne group as coupling functionality in position Z⁴.

The MC-Leu-PrgLys monomers are trimerized using the bridging molecule of structural formula BM-VIII in conjunction with the CuAAC protocol described in example 26 above to create the crosslinking entity which forms a covalent linkage between the first, second and third organic cytotoxin entity. The reaction product is isolated by semi-preparative C-18 RP-HPLC and lyophilized.

### Examples 30-33: Synthesis of homomeric microcystin trimers by crosslinking monomers of examples 26-29 with a tetrafunctional bridging moiety having an additional antibody-coupling functionality

In examples 30-33, the homomeric trimers of examples 26-29 are re-produced using different bridging molecules to create the crosslinking entity which forms the covalent linkage between the first, second and third organic cytotoxin entity. In examples 30-33, each bridging molecule carries three coupling functionalities with complementary chemical reactivity to the coupling functionalities of the microcystin monomers as described in examples 26-29, and an additional third coupling functionality configured for subsequent linkage of the trimers to an antibody or antigen-binding fragment thereof.

For this purpose, the bridging molecule BM-IX has three branches, each carrying a terminal azido group as complementary coupling functionality for crosslinking of microcystin monomers containing alkyne coupling functionalities, and a fourth branch carrying a terminal amino group as a fourth coupling functionality for linkage of the cytotoxin trimer to an antibody or antigen-binding fragment thereof:

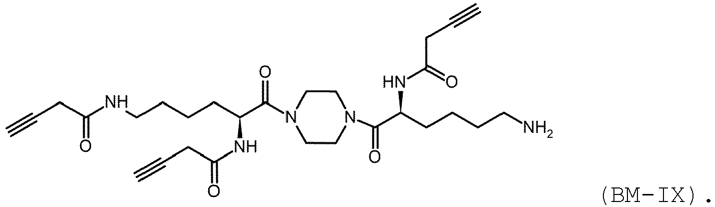

The bridging molecule BM-X has three branches, each carrying a terminal azido group as complementary coupling functionality for crosslinking of microcystin monomers containing alkyne groups as coupling functionalities, and a fourth branch carrying a terminal amino group as a fourth coupling functionality for linkage of the cytotoxin trimer to an antibody or antigen-binding fragment thereof:

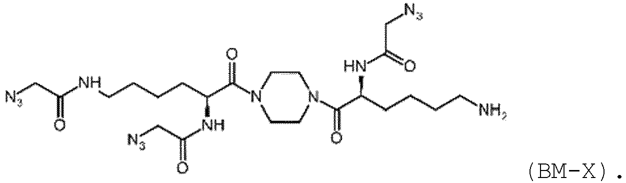

Tab. 3 provides a summary of the dimers produced in examples 12-21.

**Tab. 3: Microcystin trimers produced in examples 30-33. PrgTyr = propargyltyrosine, PrgLys = propargyllysine, AzLys = azidolysine.**

| **Example** | **Type** | **Monomer** | **Crosslinking site** | **Bridging molecule** |
|---|---|---|---|---|
| 30 | Homotrimer | MC-AzLys-Arg | X², X², X² | BM-IX |
| 31 | Homotrimer | MC-PrgTyr-Arg | X², X², X² | BM-X |
| 32 | Homotrimer | MC-Leu-AzLys | Z⁴, Z⁴, Z⁴ | BM-IX |
| 33 | Homotrimer | MC-Leu-PrgLys | Z⁴, Z⁴, Z⁴ | BM-X |

Trimerization using either the bridging molecule of structural formula BM-IX or the bridging molecule of structural formula BM-X is achieved with the CuAAC protocol described in example 26 above. The reaction products are isolated by semi-preparative C-18 RP-HPLC and lyophilized.

As an illustrative embodiment, Fig. 4 shows the structural formula TM-I of the homomeric microcystin trimer of example 30. CuAAC between the azido coupling functionalities of the MC-AzLys-Arg monomers and the complementary propargyl coupling functionalities of the bridging molecule BM-IX results in three triazole rings that covalently crosslink all three microcystin entities in position X² via the branched bridging moiety. A fourth branch of the bridging moiety carries a terminal amino group with an orthogonal reactivity to the other coupling functionalities, thereby remaining available after the trimer formation for subsequent linkage of the dimer to an antibody or antigen-binding fragment thereof.

### Example 34: Synthesis of a homomeric nodularin dimer by crosslinking monomers of [D-EdaMeAsp]¹-NOD with a bifunctional bridging moiety

A homomeric dimer derived from two [D-EdaMeAsp]¹-NOD monomers as both the first organic cytotoxin entity and the second organic cytotoxin entity is produced. The [D-EdaMeAsp]¹-NOD monomers are semi-synthetically produced using the carboxyl group of D-MeAsp as coupling functionality in position Aa¹ of naturally occurring nodularins.

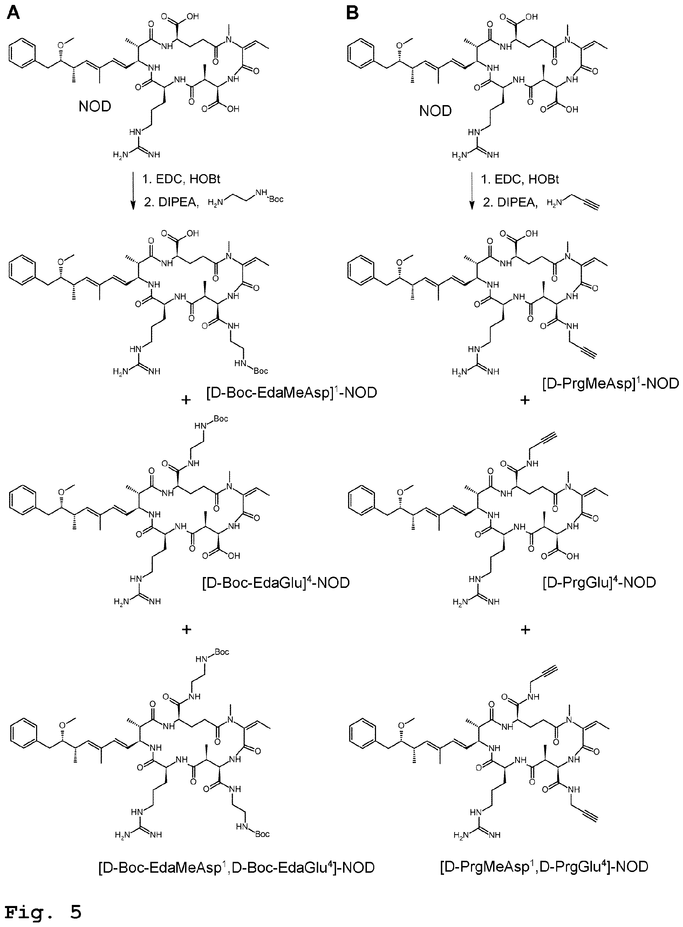

To this end, native nodularin (biosynthetically produced as described in example 1) is dissolved in DMF and stirred at 0 °C. Hydroxybenzotriazole (HOBt) (1.2 eq.) and 1-ethyl-3-(3-dimethylaminopropyl)carbodiimide (EDC) (1.2 eq.) is added. After 30 min, *N*-Boc-ethylen-1,2-diamine (Boc-Eda) (1.2 eq.) and DIPEA (2 eq.) are added to the mixture. The reaction is maintained overnight. The reaction results in a product mixture of [D-Boc-EdaMeAsp]¹-NOD and [D-Boc-EdaGlu]⁴-NOD having a protected terminal amine either in position Aa¹ or in position Aa⁴ or both [D-Boc-EdaMeAsp]¹, [D-Boc-EdaGlu]⁴-NOD as shown in Fig. 5A. The desired product [D-Boc-EdaMeAsp]¹-NOD is isolated by semi-preparative C-18 RP-HPLC and lyophilized. The Boc-protecting group is cleaved as described in example 19, yielding the [D-EdaMeAsp]¹-NOD monomer. After isolation, [D-EdaMeAsp]¹-NOD was lyophilized.

The [D-EdaMeAsp]¹-NOD monomers are dimerized using the bridging molecule BM-III for crosslinking by amide bond formation between the amino coupling functionalities in position Aa¹ of the two [D-EdaMeAsp]¹-NOD monomers and the two terminal carboxyl groups of BM-III as complementary coupling functionalities as described in example 9.

Alternatively, a similar homomeric dimer derived from two [D-MeAsp-NHS]¹-NOD carboxylic acid activated nodularin intermediates as both the first organic cytotoxin entity and the second organic cytotoxin entity is produced.

The [D-MeAsp-NHS]¹-NOD active esters are semi-synthetically produced. For this purpose, naturally occurring nodularin (described in example 1) is dissolved in DMF and stirred at 0 °C. *N*-hydroxysuccinimide (NHS) (1.2 eq.) and EDC (1.2 eq.) are added. The reaction is maintained overnight at room temperature. The reaction results in a product mixture of [D-MeAsp-NHS]¹-NOD and [D-Glu-NHS]⁴-NOD having an NHS-activated carboxylic acid in position Aa¹ or in position Aa⁴, or both [D-MeAsp-NHS]¹, [D-Glu-NHS]⁴-NOD. The desired product [D-MeAsp-NHS]¹-NOD is isolated by semi-preparative C-18 RP-HPLC and lyophilized.

The [D-MeAsp-NHS]¹-NOD active esters are dimerized using the bridging molecule of structural formula BM-XI to create the crosslinking entity which forms a covalent linkage between the first organic cytotoxin entity and the second organic cytotoxin entity. For this purpose, the bridging molecule BM-XI carries two terminal amino groups as complementary coupling functionalities:

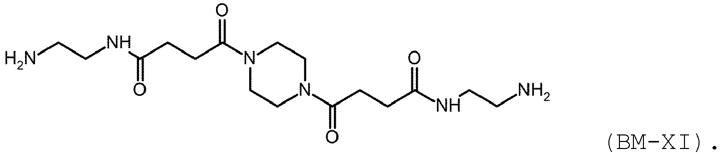

Crosslinking between the NHS activated carboxyl coupling functionalities in position Aa¹ of the two NOD monomers and the two terminal amino groups of BM-XI as complementary coupling functionalities is accomplished by amide bond formation. Briefly, [D-MeAsp-NHS]¹-NOD (2.2 eq.) and BM-XI are dissolved in DMF/sodium phosphate buffer (0.1 mM, pH 8) (1:1, v/v) and stirred at 25 °C overnight. The reaction product is isolated by semi-preparative C-18 RP-HPLC and lyophilized.

### Example 35: Synthesis of a homomeric nodularin dimer by crosslinking monomers of [D-PrgMeAsp]¹-NOD with a bifunctional bridging moiety

A homomeric dimer derived from two [D-PrgMeAsp]¹-NOD monomers as both the first organic cytotoxin entity and the second organic cytotoxin entity is produced.

The [D-PrgMeAsp]¹-NOD monomers were semi-synthetically produced as shown in Fig. 5B. To this end, naturally occurring nodularin was dissolved in DMF and stirred at 0 °C. HOBt (1.2 eq.) and EDC (1.2 eq.) was added. After 30 min, propargylamine (1.2 eq.) and DIPEA (2 eq.) was added to the mixture. The reaction was maintained overnight at 25 °C. The reaction yielded a product mixture of [D-PrgMeAsp]¹-NOD having an alkyne (propargyl) group in position Aa¹ and [D-PrgGlu]⁴-NOD having an alkyne (propargyl) group in position Aa⁴.

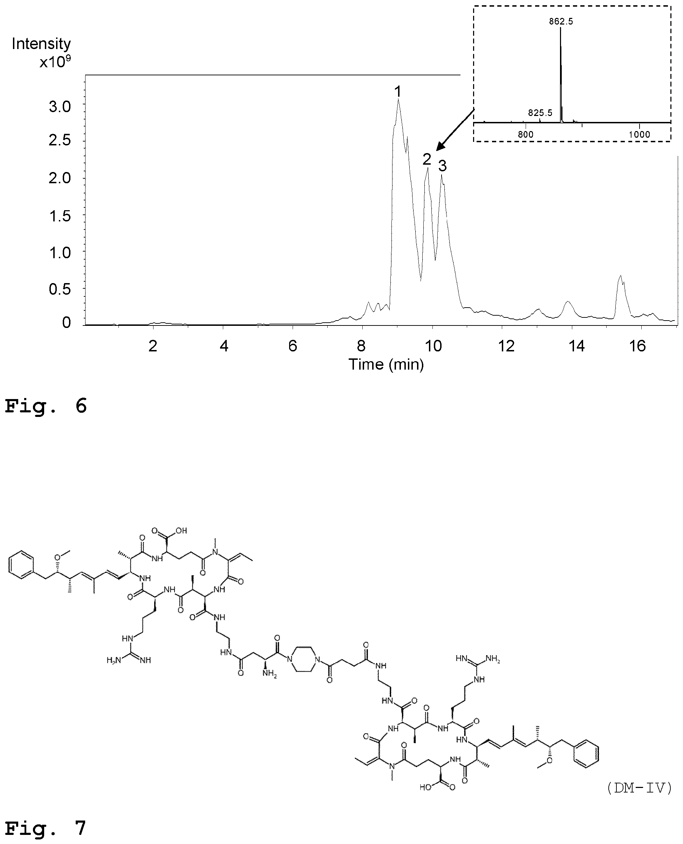

The desired product [D-PrgMeAsp]¹-NOD was then isolated by semi-preparative C-18 RP-HPLC. Fig. 6 shows a representative RP-HPLC chromatogram of the product mixture, wherein peak 1 corresponded to unmodified NOD, peak 2 corresponded to the desired product [D-PrgMeAsp]¹-NOD with an extracted-ion-chromatogramm mass of m/z = 862.5 (as shown in the insert), and peak 3 corresponded to the undesired by-product [D-PrgGlu]⁴-NOD. After isolation, [D-PrgAsp]¹-NOD was lyophilized.

The [D-PrgMeAsp]¹-NOD monomers are dimerized using the bridging molecule of structural formula BM-I in conjunction with the CuAAC protocol described in example 2 to achieve crosslinking between the alkyne coupling functionalities in position Aa¹ of the two [D-PrgMeAsp]¹-NOD monomers and the two terminal azido groups of BM-I as complementary coupling functionalities, thereby creating the crosslinking entity which forms a covalent linkage between the first organic cytotoxin entity and the second organic cytotoxin entity in the cytotoxin dimer. The reaction product is isolated by semi-preparative C-18 RP-HPLC and lyophilized.

### Example 36: Synthesis of a heteromeric nodularin dimer by crosslinking monomers of [D-PrgMeAsp]¹-NOD and [D-EdaMeAsp]¹-NOD with a difunctional bridging moiety

A heteromeric dimer derived from a [D-PrgMeAsp]¹-NOD monomer as the first organic cytotoxin entity and a [D-EdaMeAsp]¹-NOD monomer as the second organic cytotoxin entity is produced.

The [D-EdaMeAsp]¹-NOD monomer and the [D-PrgMeAsp]¹-NOD monomer were semi-synthetically produced as described in example 34 and 35 above.

The monomers [D-EdaMeAsp]¹-NOD and [D-PrgMeAsp]¹-NOD are dimerized using the bifunctional bridging molecule of structural formula BM-XII to create the crosslinking entity which forms a covalent linkage between the first organic cytotoxin entity and the second organic cytotoxin entity. For this purpose, the bifunctional bridging molecule BM-XII carries a terminal carboxylic group for conjugation with the amino group of [D-EdaMeAsp]¹-NOD via amide bond formation and a terminal azido group for CuAAC with the alkyne (propargyl) group of [D-PrgMeAsp]¹-NOD:

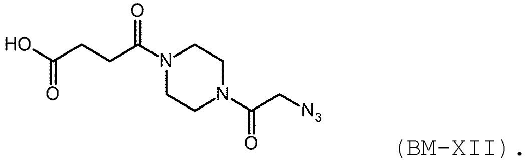

First, the D-EdaMeAsp]¹-NOD monomer is conjugated via amide bond formation of the amino group in position Aa¹ to the terminal carboxylic group of the bridging molecule BM-XII using the protocol described in example 9 with the exception that 1 eq. BM-XII, 0.95 eq. HATU, 2 eq. DIPEA and 1 eq. [D-EdaMeAsp]¹-NOD are used, thereby forming a first part of the covalent linkage of the crosslinking entity. The intermediate product is isolated by C18 RP-HPLC and lyophilized.

Afterwards, the dimerization is completed by conjugation of the [D-PrgMeAsp]¹-NOD monomer to the intermediate product by CuAAC using the protocol described in example 2, with the exception that equimolar amounts of the monomer and the intermediate product are used, to achieve crosslinking between the alkyne coupling functionality in position Aa¹ of the [D-PrgMeAsp]¹-NOD monomer and the terminal azido group of the bridging molecule BM-XII in the intermediate product as complementary coupling functionality.

In this way, the crosslinking entity is completed by forming a second part of the covalent linkage which crosslinks both nodularin entities via the crosslinking entity with each other. The dimer is isolated by C18 RP-HPLC and lyophilized.

### Examples 37-39: Synthesis of homomeric and heteromeric nodularin dimers by crosslinking monomers of examples 34-36 with a trifunctional bridging moiety having an additional antibody-coupling functionality

In examples 37-39, the homomeric and heteromeric nodularin dimers of examples 34-36 are re-produced using different bridging molecules to create the crosslinking entity which forms the covalent linkage between the first organic cytotoxin entity and the second organic cytotoxin entity. In examples 37-39, each bridging molecule carries two coupling functionalities with complementary chemical reactivity to the coupling functionalities of the nodularin monomers as described in examples 34-36, and an additional third coupling functionality configured for subsequent linkage of the dimers to an antibody or antigen-binding fragment thereof.

For this purpose, the bridging molecule BM-VI described in example 19 carries two carboxylic groups as complementary coupling functionalities for crosslinking of [D-EdaMeAsp]¹-NOD monomers containing amine coupling functionalities and a terminal protected amino group. Subsequent to the dimerization reaction, the protection group is cleaved as described in example 19, revealing a free amino group for linkage of the dimer to an antibody or antigen-binding fragment thereof.

In an alternative synthesis route, the bridging molecule BM-XIII is used, which carries two free amino groups and one Boc-protected amino group. The two amino groups are used as a first and a second complementary coupling functionality for crosslinking of the [D-MeAsp-NHS]¹-NOD monomers via the activated carboxyl groups as coupling functionalities. After dimerization, the Boc-group is cleaved as described in example 19 revealing a free amino group as a third coupling functionality for linkage of the dimer to an antibody or antigen-binding fragment thereof:

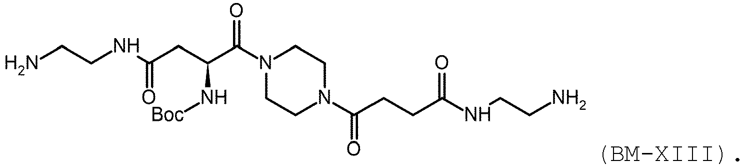

The bridging molecule BM-IV described in example 12 carries two azido groups as complementary coupling functionalities for crosslinking of [D-PrgMeAsp]¹-NOD monomers containing alkyne coupling functionalities and a terminal amino group as a third coupling functionality for linkage of the dimer to an antibody or antigen-binding fragment thereof.

In addition, the bridging molecule BM-XIV carries one azido group as a complementary coupling functionality for crosslinking of [D-PrgMeAsp]¹-NOD monomers containing alkyne coupling functionalities and a terminal carboxyl group for conjugation to the amino group of the [D-EdaMeAsp]¹-NOD monomer. Moreover, BM-XIV carries a terminal Boc-protected amino group as a third coupling functionality. After dimerization, the Boc-group is cleaved as described in example 19, revealing a free amino group as a third coupling functionality for linkage of the heterodimer to an antibody or antigen-binding fragment thereof:

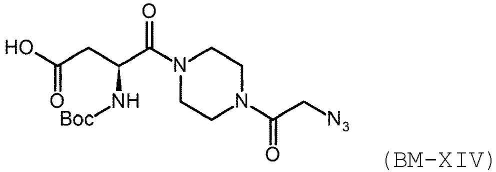

Table 4 provides a summary of the dimers produced in examples 37-39.

**Table 4: Nodularin (NOD) dimers produced in examples 37-39. D-PrgMeAsp = D-erythro-β-propargylamine-β-methylaspartic acid.**

| **Example** | **Type** | **Monomer** | **Crosslinking site** | **Bridging molecule** |
|---|---|---|---|---|
| 37 | Homodimer | [D-EdaMeAsp]¹-NOD or [D-MeAsp-NHS]¹-NOD | Aa¹-Aa¹ | BM-VI or BM-XIII |
| 38 | Homodimer | [D-PrgMeAsp]¹-NOD | Aa¹-Aa¹ | BM-IV |
| 39 | Heterodimer | [D-PrgMeAsp]¹-NOD, [D-EdaMeAsp]¹-NOD | Aa¹-Aa¹ | BM-XIV |

Dimerization using the bridging molecule of structural formula BM-VI or BM-XIII is achieved with the protocol described for the bridging molecule of structural formula BM-III or BM-XI in the corresponding example 34. Dimerization using the bridging molecule of structural formula BM-IV is achieved with the CuAAC protocol described in example 2 and in the corresponding example 35. Dimerization using the bridging molecule of structural formula BM-XIV is achieved with the protocol described for the bridging molecule of structural formula BM-XII in the corresponding example 36.

The reaction products are isolated by semi-preparative C-18 RP-HPLC and lyophilized.

As an illustrative embodiment, Fig. 7 shows the structural formula DM-IV of the homomeric nodularin dimer of example 37. As described above, the conjugation reaction is achieved either between the amino groups of the [D-EdaMeAsp]¹-NOD and the complementary carboxyl groups of bridging molecule BM-VI or between the activated carboxyl coupling functionalities of the [D-MeAsp-NHS]¹-NOD monomers and the complementary amino coupling functionalities of the bridging molecule BM-XIII. In both ways, two amide bonds are formed that covalently crosslink both nodularin entities in position Aa¹ via two opposite branches of the bridging moiety. Furthermore, the bridging molecule contains an additional amino group substituent remaining available after the dimer formation for subsequent linkage of the dimer to an antibody or antigen-binding fragment thereof.

### Example 40: Synthesis of a homomeric nodularin trimer by crosslinking monomers of [D-PrgMeAsp]¹-NOD with a trifunctional bridging moiety

A homomeric trimer derived from three [D-PrgMeAsp]¹-NOD monomers described in example 34 as the first, second and third organic cytotoxin entity is produced. The [D-PrgMeAsp]¹-NOD monomers have an alkyne group as coupling functionality in position Aa¹.

The [D-PrgMeAsp]¹-NOD monomers are trimerized using the bridging molecule of structural formula BM-VIII in conjunction with the CuAAC protocol described in example 26 above to create the crosslinking entity which forms a covalent linkage between the first, second and third organic cytotoxin entity. The reaction product is isolated by semi-preparative C-18 RP-HPLC and lyophilized.

### Example 41: Synthesis of a homomeric nodularin trimer by crosslinking monomers of [D-PrgMeAsp]¹-NOD with a tetrafunctional bridging moiety having an additional antibody-coupling functionality

A homomeric nodularin trimer derived from three [D-PrgMeAsp]¹-NOD monomers described in example 34 as the first, second and third organic cytotoxin entity is produced. The [D-PrgMeAsp]¹-NOD monomers have an alkyne group as coupling functionality in position Aa¹.

The [D-PrgMeAsp]¹-NOD monomers are trimerized using the bridging molecule of structural formula BM-X in conjunction with the CuAAC protocol described in example 26 above to create the crosslinking entity which forms a covalent linkage between the first, second and third organic cytotoxin entity via three branches of the bridging molecule BM-X. The reaction product is isolated by semi-preparative C-18 RP-HPLC and lyophilized.

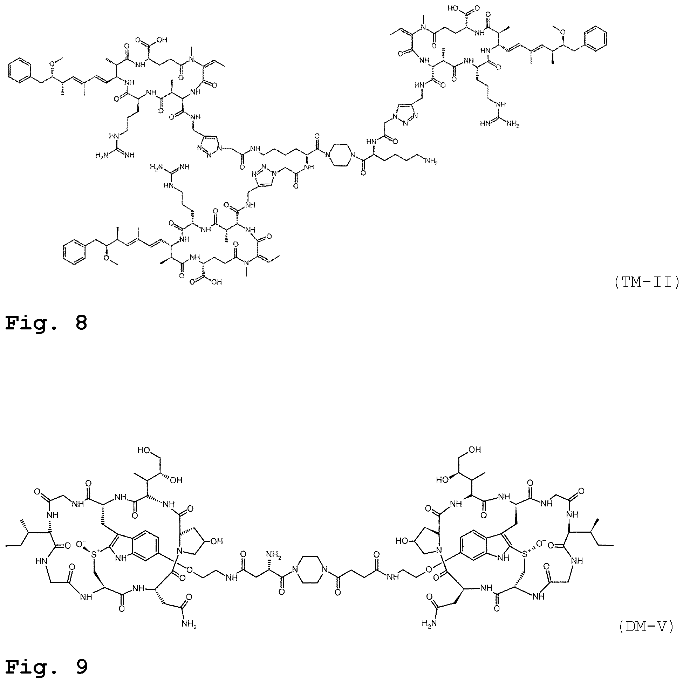

As an illustrative embodiment, Fig. 8 shows the structural formula TM-II of the homomeric nodularin trimer of example 41. CuAAC between the alkyne coupling functionalities of the [D-PrgMeAsp]¹-NOD monomers and the complementary azido coupling functionalities of the bridging molecule BM-X results in three triazole rings that covalently crosslink all three microcystin entities in position Aa¹ via the branched bridging moiety. A fourth branch of the bridging moiety carries a terminal amino group with an orthogonal reactivity to the other coupling functionalities, thereby remaining available after the trimer formation for subsequent linkage of the dimer to an antibody or antigen-binding fragment thereof.

### Example 42: Synthesis of a homomeric amatoxin dimer by crosslinking monomers of [AeHtp]²-AMA with a bifunctional bridging moiety

A homomeric dimer derived from two [AeHtp]²-AMA monomers (SEQ ID NO:14) as both the first organic cytotoxin entity and the second organic cytotoxin entity is produced.

The [AeHtp]²-AMA monomers are semi-synthetically produced using a hydroxyindol group as coupling functionality in position Aa² of naturally occurring AMA described in example 1.

The [AeHtp]²-AMA monomer is produced in two steps. First, the hydroxyindol groups in position Aa² is etherified with alkyl halide 2-(Boc-amino)ethyl bromid (Boc-Ae-Br) using the etherification protocol for amanitin derivatization of Faulstich et al. (Biochemistry 1981, 20, 6498-504). Briefly, native AMA is dissolved in dry ethanol. Freshly prepared sodium ethylate solution (1.1 eq.) is added and the solution is afterwards directly evaporated. The remaining solid and Boc-Ae-Br (4 eq.) are dissolved in DMF and stirred overnight at 20 °C. The reaction results in a product mixture of the monoalkylated [Boc-AeHtp]²-AMA, the dialkylated [(Boc-Ae)₂Htp]²-AMA and unmodified native AMA. The desired product [Boc-AeHtp]²-AMA is isolated by semi-preparative C-18 RP-HPLC and lyophilized. In the second step, the Boc-group is cleaved as described in example 19, thereby yielding the [AeHtp]²-AMA monomer.

Crosslinking between the amino coupling functionalities in position Aa² of the two [AeHtp]²-AMA-1 monomers and the two terminal carboxyl groups of BM-III as complementary coupling functionalities is accomplished by amide bond formation as described in example 9.

### Example 43: Synthesis of a homomeric amatoxin dimer by crosslinking monomers of [PrgDhil]¹-AMA with a bifunctional bridging moiety

A homomeric dimer derived from two [PrgDhil]¹-AMA monomers (SEQ ID NO:15) as both the first organic cytotoxin entity and the second organic cytotoxin entity is produced.

The [PrgDhil]¹-AMA monomers were semi-synthetically produced using a primary hydroxyl group as coupling functionality in position Aa¹ of naturally occurring AMA. First, native AMA is dissolved in dry DMF and stirred at 0 °C under nitrogen. Then, bis(para-nitrophenyl(bis-PNP)) carbonate (1.2 eq.) and DIPEA (1.2 eq.) are added and the reaction mixture is stirred for 2-24 h at 0 °C. After evaporation of DMF, the residue is triturated in diethyl ether and the precipitate is isolated by filtration to yield the AMA-PNP intermediate. Then, propargylamine (2 eq.) is dissolved in DMF and AMA-PNP (1 eq.) and DIPEA (2 eq.) are added to the solution and stirred for 2-24 h at 20 °C. The reaction product [PrgDhil]¹-AMA is isolated by semi-preparative C-18 RP-HPLC and lyophilized.

The [PrgDhil]¹-AMA monomers are dimerized using the bridging molecule of structural formula BM-I in conjunction with the CuAAC protocol described in example 2 to create the crosslinking entity which forms a covalent linkage between the first organic cytotoxin entity and the second organic cytotoxin entity. The reaction product is isolated by semi-preparative C-18 RP-HPLC and lyophilized.

### Example 44: Synthesis of a heteromeric amatoxin dimer by crosslinking monomers of [AeHtp]²-AMA and [PrgDhil]¹-AMA with a bifunctional bridging moiety

A heteromeric dimer derived from [AeHtp]²-AMA (SEQ ID NO:15) as the first organic cytotoxin entity and [PrgDhil]¹-AMA as the second organic cytotoxin entity is produced. The [AeHtp]²-AMA monomer and [PrgDhil]¹-AMA monomer are semi-synthetically produced as described in example 42 and example 43 above.

The monomers of [AeHtp]²-AMA and [PrgDhil]¹-AMA are dimerized using the bifunctional bridging molecule of structural formula BM-XII to create the crosslinking entity which forms a covalent linkage between the first organic cytotoxin entity and the second organic cytotoxin entity. For this purpose, the bifunctional bridging molecule BM-XII carries a terminal carboxylic group for conjugation with the amino group of [AeHtp]²-AMA via amide bond formation and a terminal azido group for CuAAC with the alkyne (propargyl) group of [PrgDhil]¹-AMA using the protocol described in example 36.

### Examples 45-47: Synthesis of homomeric and heteromeric amatoxin dimers by crosslinking monomers of examples 42-44 with a trifunctional bridging moiety having an additional antibody-coupling functionality

In examples 45-47, the homomeric and heteromeric amatoxin dimers of examples 42-44 are re-produced using a different bridging molecule to create the crosslinking entity which forms the covalent linkage between the first organic cytotoxin entity and the second organic cytotoxin entity. In examples 45-47, each bridging molecule carries two coupling functionalities with complementary chemical reactivity to the coupling functionalities of the amanitin monomers as described in examples 42-44, and an additional third coupling functionality configured for subsequent linkage of the dimers to an antibody or antigen-binding fragment thereof.

**Tab. 5: Amatoxin (AMA) dimers produced in examples 45-47.**

| **Example** | **Type** | **Monomer** | **Crosslinking site** | **Bridging molecule** |
|---|---|---|---|---|
| 45 | Homodimer | [AeHtp]²-AMA | Aa²-Aa² | BM-VI |
| 46 | Homodimer | [PrgDhil]¹-AMA | Aa¹-Aa¹ | BM-IV |
| 47 | Heterodimer | [AeHtp]²-AMA, [PrgDhil]¹-AMA | Aa²-Aa¹ | BM-XIV |

Dimerization using the bridging molecule of structural formula BM-VI is achieved with the protocol described for the bridging molecule of structural formula BM-III in the corresponding example 42. Dimerization using the bridging molecule of structural formula BM-IV is achieved with the CuAAC protocol described in example 2 and in the corresponding example 43. Dimerization using the bridging molecule of structural formula BM-XIV is achieved with the protocol described for the bridging molecule of structural formula BM-XII in the corresponding example 44.

The reaction products are isolated by semi-preparative C-18 RP-HPLC and lyophilized.

As an illustrative embodiment, Fig. 9 shows the structural formula DM-V of the homomeric amatoxin dimer of example 45. The conjugation reaction between the amino coupling functionalities of the [AeHtp]²-AMA monomers and the complementary carboxyl coupling functionalities of the bridging molecule BM-VI forms two amide bonds that covalently crosslink both amatoxin entities in position Aa² via two opposite branches of the bridging moiety. After Boc-deprotection subsequent to dimerization, the bridging molecule contains an additional amino group substituent available for subsequent linkage of the dimer to an antibody or antigen-binding fragment thereof.

### Example 48: Synthesis of a homomeric amatoxin trimer by crosslinking monomers of [AeHtp]²-AMA with a trifunctional bridging moiety

A homomeric trimer derived from three [AeHtp]²-AMA monomers (SEQ ID NO:14) described in example 42 as the first, second and third organic cytotoxin entity is produced. The [AeHtp]²-AMA monomers have an amino group as coupling functionality in position Aa².

The [AeHtp]²-AMA monomers are trimerized using the bridging molecule of structural formula BM-XV:

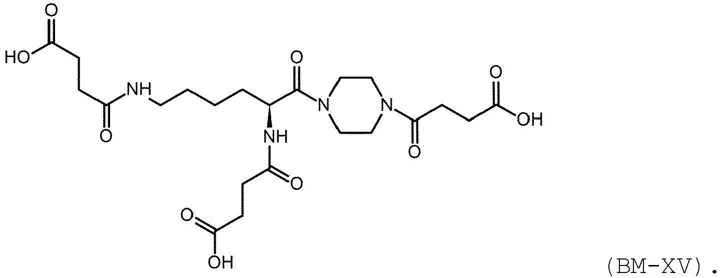

Trimerization using the bridging molecule of structural formula BM-XV is achieved by amide bond formation between the amino groups of the [AeHtp]²-AMA monomers and the carboxyl groups of the bridging molecule using the protocol described in example 43 above with adjusted 3 eq. of the [AeHtp]²-AMA monomer, 1 eq. BM-XV, 2.95 eq. HATU and 6 eq. DIPEA to create the crosslinking entity which forms a covalent linkage between the first, second and third organic cytotoxin entity.

The reaction product is isolated by semi-preparative C-18 RP-HPLC and lyophilized.

### Example 49: Synthesis of a homomeric amatoxin trimer by crosslinking monomers of [PrgDhil]¹-AMA with a trifunctional bridging moiety

A homomeric trimer derived from three [PrgDhil]¹-AMA monomers (SEQ ID NO:15) as the first, second and third organic cytotoxin entity is produced. The [PrgDhil]¹-AMA monomers have an alkyne group as coupling functionality in position Aa¹.

The [PrgDhil]¹-AMA monomers are trimerized using the bridging molecule of structural formula BM-VIII as described in example 27 above. The reaction product is isolated by semi-preparative C-18 RP-HPLC and lyophilized.

### Examples 50-51: Synthesis of homomeric amatoxin trimers by crosslinking monomers of examples 48-49 with a trifunctional bridging moiety having an additional antibody-coupling functionality

In examples 50 and 51, the homomeric amatoxin trimers of examples 48 and 49 are re-produced using a different bridging molecule to create the crosslinking entity which forms the covalent linkage between the first, second and third organic cytotoxin entity. In examples 50, the bridging molecule of structural formula BM-XVI is used:

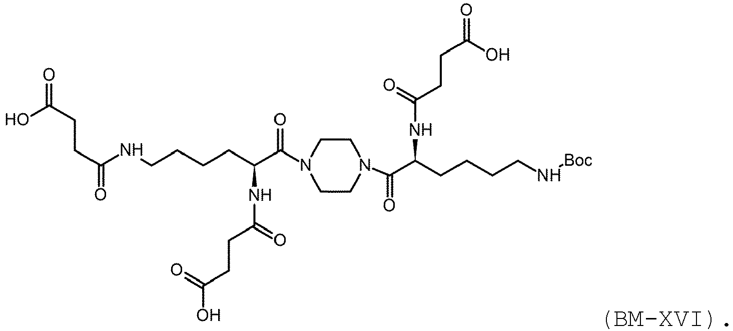

Accordingly, the bridging molecule BM-XVI carries three carboxyl groups as coupling functionalities with complementary chemical reactivity to the amino coupling functionalities of the amatoxin monomers as described in example 48, and a Boc-protected amino group as additional fourth coupling functionality configured for subsequent linkage of the cytotoxin trimers to an antibody or antigen-binding fragment thereof.

**Tab. 6: Amatoxin (AMA) trimers produced in examples 50 and 51.**

| **Example** | **Type** | **Monomer** | **Crosslinking site** | **Bridging molecule** |
|---|---|---|---|---|
| 50 | Homotrimer | [AeHtp]²-AMA | Aa²-Aa² | BM-XVI |
| 51 | Homotrimer | [PrgDhil]¹-AMA | Aa¹-Aa¹ | BM-X |

Trimerization using the bridging molecule of structural formula BM-XVI is achieved by amide bond formation between the amino groups of the [AeHtp]²-AMA monomers and the carboxyl groups of the bridging molecule using the protocol described in example 48 to create the crosslinking entity which forms a covalent linkage between the first, second and third organic cytotoxin entity. Subsequent to the dimerization reaction, the Boc-group is cleaved as described in example 19. The reaction product is isolated by semi-preparative C-18 RP-HPLC and lyophilized.

Trimerization using the bridging molecule of structural formula BM-X is achieved by CuAAC with the protocol described for the bridging molecule of structural formula BM-X in the corresponding example 26.

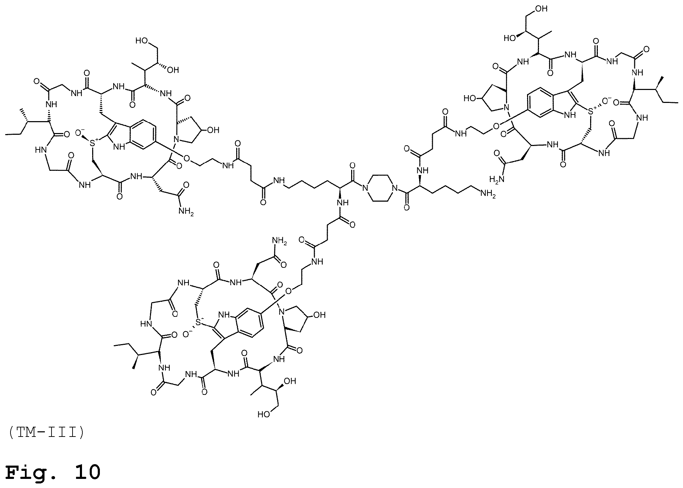

As an illustrative embodiment, Fig. 10 shows the structural formula TM-III of the homomeric amatoxin trimer of example 50. The conjugation reaction between the terminal amino group of the [AeHtp]²-AMA monomers and the complementary carboxyl coupling functionalities of the bridging moiety BM-XVI forms three amide bonds that covalently crosslink the three amatoxin entities in position Aa² via different branches of the bridging moiety. Furthermore, the bridging molecule contains an additional Boc-protected amino group substituent remaining available after the trimer formation and subsequent Boc-deprotection for linkage of the trimer to an antibody or antigen-binding fragment thereof.

### Example 52: Synthesis of an ADC composed of a monoclonal anti-HER2 antibody and the organic cytotoxin dimer of example 12 as drug moiety

The organic cytotoxin dimer synthesised in example 12 is used as the drug moiety in the construction of an ADC. The organic cytotoxin dimer is a microcystin homodimer consisting of two MC-PrgTyr-Arg cytotoxin entities that are symmetrically crosslinked in position X² via two branches of the bridging moiety BM-IV. The bridging moiety further comprises a free amino coupling functionality for attachment of the microcystin homodimer to an antibody. Fig. 2 shows the corresponding structural formula DM-II.

The monoclonal antibody (mAb) trastuzumab (brand name "Herceptin") targets human epidermal growth factor receptor 2 (Her2, also known as ERBB2, erb-b2 receptor tyrosine kinase 2). For production of the mAb, genes encoding the heavy and light chain variable regions of trastuzumab are synthesized and cloned into human heavy chain and light chain vectors encoding human IgG1. The sequence of the constructs is verified by DNA sequencing. The vectors are transiently transfected and expressed in CHO cells. The mAb is purified from cell culture supernatant (e. g. using Protein A) to a purity of more than 90%. The purified mAb is quantified by absorption at 280 nm and characterized by SEC and SDS-PAGE. It is understood that the protocol can be adapted using genes encoding heavy and light chain variable regions of other monoclonal antibodies to produce ADCs having other target specificities without departing from the teaching of this invention.

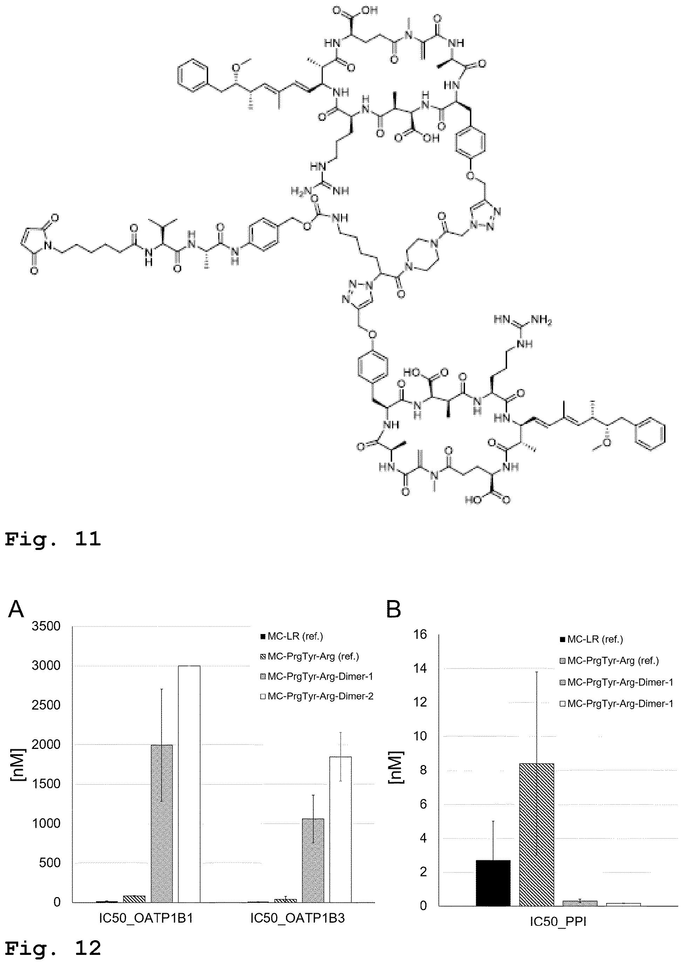

The microcystin homodimer obtained according to example 12 was conjugated to a cleavable linker maleimidocaproyl-Val-Ala-(p-amino-benzyl)-(para-nitro-phenyl)-carbonate (mc-Val-Ala-PAB-PNP) by incubation of 1 eq. of the microcystin dimer and 1 eq. of the linker in DMF and DIPEA (2 eq.) at 25 °C for 1-3 hours. In this way, the microcystin dimer was coupled to the linker through carbamate-bond formation between an activated carbonate on the linker and the free amino group in the bridging moiety of the microcystin dimer yielding mc-Val-Ala-PABC-DII (PABC = p-amino-benzyl carbamate) hereinafter termed "DM-II-L". The obtained linker-microcystin dimer conjugate was isolated by RP-HPLC and characterized for identity (MS) and purity (RP-LC) aiming at a purity of at least 95%. Fig. 11 shows the structural formula of the linker-microcystin dimer conjugate DM-II-L. Other suitable linkers and conjugation chemistries are known to the skilled artisan.

Subsequently, the resulting linker-homodimer conjugate was coupled to the monoclonal anti-HER2 antibody trastuzumab (brand name: Herceptin), a representative benchmark antibody of the applied art, using complementary coupling functionalities of the monoclonal antibody and the linker of the linker-homodimer conjugate to form the ADC. For this purpose, native (non-engineered) mAb was dissolved in 5% tris(hydroxymethyl)-aminomethane (TRIS) buffer supplemented with 25 mM ethylenediaminetetraacetic acid (EDTA), pH 8.5 to a final concentration of 26.5 mg/mL. For mAb reduction, 1 eq. tris(2-carboxyethyl)phosphine hydrochloride (TCEP, 10 mM) for a final DAR of about 2, or 2 eq. for a final DAR of about 4 was added. The reaction was incubated for 1.5 at room temperature, followed by dilution with 2 mM EDTA in PBS (pH 7.4) to 5 mg/mL.

The buffer was exchanged and desalted by gel filtration (Zeba^{™} spin) with PBS containing 2 mM EDTA for a DAR of 2, additionally supplemented with 3% cyclodextrin (CD) for a DAR of 4 to reduce antibody aggregation. For conjugation of the linker-microcystin dimer conjugate to the reduced mAb, 4 eq. of DM-II-L for a DAR of about 2, taken from a 10 mM DM-II-L solution in dimethylacetamide (DMA), was added to the reduced mAb (5 mg/mL, PBS with 2 mM EDTA), and the reaction was incubated for 2 h at room temperature. For a DAR of about 4, 8 eq. of DM-II-L was used and added to the reduced mAb (5 mg/mL, 3% CD in PBS with 2 mM EDTA) and incubated for 3.5 h at room temperature. For better solubility, a final concentration of 5% DMA was used for DAR 2 and 10% DMA (v/v) for DAR 4. The conjugation reaction was quenched for 30 min by addition of an equimolar amount of N-Acetyl-L-Cysteine (NAC) in relation to the used toxin-linker conjugate. Then, the reaction mixture was desalted by gel filtration (G-25, PBS (DAR 2) or 3% CD in PBS (DAR 4)). Afterwards, activated carbon (100 mg/mL in PBS or 3% CD in PBS pH 7.4), in an equal amount to the weight of mAb was added. After 1 hour, activated carbon was removed by centrifugation (4000 g, 15 min), the supernatant was analyzed by SEC and HIC and sterile filtered.

### Example 53: Synthesis of an ADC composed of a monoclonal anti-HER2 antibody and the organic cytotoxin dimer of example 18 as drug moiety

The organic cytotoxin dimer synthesised in example 18 is used as the drug moiety in the construction of an ADC. The organic cytotoxin dimer is a microcystin homodimer consisting of two MC-Leu-AzLys entities that are symmetrically crosslinked in position Z⁴ via two branches of the bridging moiety BM-V. The bridging moiety further comprises a free amino coupling functionality for attachment of the microcystin homodimer to an antibody.

First, the microcystin homodimer obtained according to example 18 is conjugated to a cleavable linker such as mc-Val-Ala-PAB-PNP via the free terminal amino group of the bridging moiety BM-V, as described in example 52 above. Other suitable linkers and conjugation chemistries are known to the skilled artisan.

Subsequently, the resulting linker-homodimer conjugate is coupled to the monoclonal anti-HER2 antibody trastuzumab as described in example 52 above.

### Example 54: Synthesis of an ADC composed of a monoclonal anti-HER2 antibody and the organic cytotoxin dimer of example 38 as drug moiety

The organic cytotoxin dimer synthesised in example 38 is used as the drug moiety in the construction of an ADC. The organic cytotoxin dimer is a nodularin homodimer consisting of two [D-PrgMeAsp]¹-NOD entities that are symmetrically crosslinked in position Aa¹ via two branches of the bridging moiety BM-IV. The bridging moiety further comprises a free amino coupling functionality for attachment of the nodularin homodimer to an antibody.

First, the nodularin homodimer obtained according to example 38 is conjugated to a cleavable linker such as mc-Val-Ala-PAB-PNP via the free terminal amino group of the bridging moiety BM-IV as described in example 52 above. Other suitable linkers and conjugation chemistries are known to the skilled artisan.

Subsequently, the resulting linker-homodimer conjugate is coupled to the monoclonal anti-HER2 antibody trastuzumab as described in example 52 above.

### Example 55: Synthesis of an ADC composed of a monoclonal anti-HER2 antibody and the organic cytotoxin dimer of example 45 as drug moiety

The organic cytotoxin dimer synthesised in example 45 is used as drug moiety in the construction of an ADC. The organic cytotoxin dimer is an amatoxin homodimer consisting of two [AeHtp]²-AMA cytotoxin entities that are symmetrically crosslinked in position Aa² via two branches of the bridging moiety BM-VI. The bridging moiety further comprises a free amino coupling functionality for attachment of the microcystin homodimer to an antibody (see Fig. 9).

First, the amatoxin homodimer obtained according to example 45 is conjugated to a cleavable linker such as mc-Val-Ala-PAB-PNP via the free terminal amino group of the bridging moiety BM-VI as described in example 53. Other suitable linkers and conjugation chemistries are known to the skilled artisan.

Subsequently, the resulting linker-homodimer conjugate is coupled to the monoclonal anti-HER2 antibody trastuzumab using complementary coupling functionalities of the monoclonal antibody and the linker of the linker-homodimer conjugate as described in example 52 above to form the ADC.

### Example 56: Protocol for cell culture of OATP-expressing cancer cell lines and subsequent cytotoxicity assay

HEK293 cells, stably transfected with expression vectors pcDNA3.1(+)-OATPB1 and pcDNA3.1/Hygro(-)-OATP1B3, and the respective empty vectors pcDNA3.1(+) and pcDNA3.1/Hygro(-) as controls were kindly provided by Prof. Dr. Jörg König (Friedrich-Alexander-University Erlangen-Nürnberg, Germany).

All cell lines were maintained in minimal essential medium, supplemented with 10% heat-inactivated fetal bovine serum, non-essential amino acid mix and 2 mM glutamine at 37 °C and 5% CO₂.

HEK293 OATP1B1+ and the corresponding control cell line were constantly selected with 800 ug/mL geneticin (G418), while 250 ug/mL of hygromycin B were used for the selection of HEK293 OATP1B3+ and its empty vector control.

To determine the cytotoxicity of the organic cytotoxin oligomers of the present invention and corresponding cytotoxin monomers as reference, 5 x 10⁴ cells per well of each cell line were seeded in 96-well plates in triplicates without selection marker. After 24 hours, transporter expression was induced by addition of sodium butyrate to a final concentration of 10 mM. On the next day, the medium was removed, and the cells were incubated with eight different concentrations of each of the cytotoxin oligomers (0.01 nM to 3 µM) and corresponding toxin monomers as controls in medium without selection markers for 48 hours.

Then, the cells were fixed, washed and stained with sulforhodamine B (SRB) as previously described (Vichai et al., Nature protocols 2006, 1, 1112-1116). In brief, 10 µL trichloroacetic acid (10%) were directly added to each well and incubated for 1 h at 4 °C. Then, the solution was removed and each well washed thrice with 200 µL H₂O. Afterwards, the wells were dried and 100 µL 0.057% SRB-solution (in 1% acetic acid) was added per well and incubated for 30 minutes at 4 °C. The solution was removed and each well was washed thrice with 200 µL acetic acid (1%). After drying of the wells, 200 µL Tris buffer (10 mM, pH 10.5) was added and the plates were shaked for five minutes. Then, the absorbance of SRB was measured at 510 nm with a TECAN Infinity TECAN Infinity M PLEX plate reader (Tecan Deutschland GmbH, Crailsheim, Germany). Experiments were performed at least in duplicate.

The cell viability and the IC50 values (IC50_Cytotox) were calculated with GraphPad PRISM 6 using a nonlinear regression (sigmoidal dose-response).

### Example 57: Protocol for protein phosphatase inhibition (PPI) assay

The PPI assay was carried out as recently described (Heresztyn et al. Water Res 2001, 35, 3049-3056).

Prior to the measurement, different working solutions have been prepared. The reaction buffer was prepared freshly by combining Tris buffer (250 mM, pH 8.1), magnesium chloride solution (200 mM), manganese chloride solution (10 mM) and bovine serum albumin (BSA) solution (5 mg/ml) (50:26:4:20 (v/v)) and stored on ice. For the substrate solution, the reaction buffer was mixed with para-nitrophenyl phosphate (pNPP) stock solution (60 mM) and DTT solution (20 mM) (5:4:1, (v/v)), wherein the pNPP was freshly prepared. The enzyme dilution buffer was obtained by combining solutions of ethylene glycol-bis-(2-aminoethyl)-N,N,N',N'-tetraacetic acid (EGTA; 0.19 mM, in Tris buffer (80 mM, pH 7.0)), manganese chloride (10 mM), dithiothreitol (DTT; 20 mM) and BSA (5 mg/ml) (65:20:5:10 (v/v)). For the enzyme solution, the protein phosphatase-1 catalytic subunit (a-Isoform from rabbit, Sigma-Aldrich) was diluted with enzyme dilution buffer to 10 units/mL and stored on ice.

The assay was performed in 384-well plates. The diluted organic cytotoxin oligomers and the corresponding cytotoxin monomers as controls (4 µL in DMSO) were preincubated with enzyme solution (4 µL) at 37 °C for 5 minutes. Then, 40 µL substrate solution were added to each well and incubated at 37 °C for 2 hours. The seven cytotoxin concentrations range from 0.001 nM to 1 µM, while a constant substrate concentration of 20 mM was used per well. As positive control, 4 µL DMSO were used instead of MC solution to follow substrate conversion without PPI. The negative control (blank) contained 4 µL enzyme dilution buffer instead of the diluted enzyme preventing any hydrolysis of the substrate. To determine the PPI, the absorbance was measured at 405 nm with a TECAN Infinity M PLEX plate reader. Experiments were performed at least in duplicate and the IC50 values (IC50_PPI) were calculated with GraphPad PRISM 6 using a nonlinear regression (sigmoidal dose-response).

### Example 58: Protocol for in-vitro testing of ADC efficacy

The constructed ADCs of examples 52-55 are tested for efficacy in-vitro using cell viability assays in which a tumor antigen-expressing cancer cell line such as NCI-N87 expressing the Her2 antigen is incubated with different concentrations of each ADC in a picomolar to nanomolar or micromolar range.

The in-vitro efficacy testing of the ADC is performed using e. g. the CellTiter-Glo Luminescent Cell Viability Assay (Promega GmbH, Walldorf, Germany) according to the manufacturer's instructions.

### Example 59: Bioassay results

As a representative example, Fig. 12 and Tab. 7 show the results derived from the assays described in Examples 56 and 57 above in terms of the general cytotoxicity on the OATP1B1 and OATP1B3 expressing cell lines (IC50_OATP1B1 and IC50_OATP1B3 in Fig. 12 A) and the protein phosphatase inhibition assays (IC50_PPI in Fig. 12 B) for the microcystin homodimers of example 2 and example 12 with the structural formula DM-I and DM-II (see Fig. 1 and Fig. 2), which were derived from covalent crosslinking of two MC-PrgTyr-Arg monomers (SEQ ID NO:2) as the first and second organic cytotoxin entity using the bridging molecule of structural formula BM-I (hereinafter termed "MC-PrgTyr-Arg-Dimer-1") and BM-IV (hereinafter termed "MC-PrgTyr-Arg-Dimer-2") .

As a reference, Fig. 12 and Tab. 7 also show the corresponding assay results for the unconjugated reference monomer MC-PrgTyr-Arg as well as for MC-LR, which is one of the most potent and the best studied microcystin analogue currently known in the art having the highest protein phosphatase inhibition described in the literature.

**Tab. 7: Results from cytotoxicity assays with OATP1B1 expressing cell lines (IC50_OATP1B1) and OATP1B3 expressing cell lines (IC50_OATP1B3) as well as protein phosphatase inhibition assays (IC50_PPI = type 1 protein phosphatases) for MC-LR (reference), the MC-PrgTyr-Arg monomer (reference) and the corresponding MC-PrgTyr-Arg-dimers according to the present invention (examples 2 and 12). "SE" denotes standard error of at least duplicate experiments.**

| Organic cytotoxin | IC50_OATP1B1 ± SE [nM] | IC50_OATP1B3 ± SE [nM] | IC50_PPI ± SE [nM] | Ratio of IC50_OATP1B1/ IC50_PPI | Ratio of IC50_OATP1B3/ IC50_PPI |
|---|---|---|---|---|---|
| MC-LR (ref.) | 17.1 ± 2.1 | 7.9 ± 1.7 | 2.7 1 2.3 | 6.3 | 2.9 |
| MC-PrgTyr-Arg (ref.) | 82.2 ± 4.5 | 40.4 ± 36.6 | 8.4 ± 5.4 | 9.8 | 4.8 |
| MC-PrgTyr-Arg-Dimer-1 (example 2) | 1996 ± 711 | 1060 ± 302 | 0.29 ± 0.14 | 6883 | 3655 |
| MC-PrgTyr-Arg-Dimer-2 (example 12) | >3000* | 1849 ± 309 | 0.16 ± 0.02 | >18750 | 11556 |
| *no dose-response obtained by the applied concentrations up to the highest concentration of 3000 nM | | | | | |

Tab. 7 also shows ratio of the IC50_OATP1B1 and the IC50_PPI as well as the ratio of the IC50_OATP1B3 and the IC50_PPI as a measure of the PPI-normalized OATP uptake. These ratios were used to evaluate whether the oligomerization shows the desired effect. In this context, if the general cytotoxicity on the OATP1B1 and/or OATP1B3 expressing cell lines decreases (reflected by an increased IC50_OATP1B1 or increased IC50_OATP1B3, respectively) while the protein phosphatase inhibition increases or remains essentially unchanged (reflected by an unchanged or decreased IC50_PPI), i. e. the ratio is greater than the ratio of the reference, the therapeutic window of the organic cytotoxin oligomer of the present invention is enhanced with respect to the tested OATP-expressing cell line. Such enhancement of the therapeutic window occurs, as the active cell uptake of the organic cytotoxin oligomer of the present invention was reduced relative to the protein phosphatase inhibition and/or because the protein phosphatase inhibition of the organic cytotoxin oligomer of the present invention was increased relative to the active cell uptake. Furthermore, if the ratio of IC50_PPI of the organic cytotoxin oligomer of the present invention and the IC50_PPI of the references is < 0.5 in case the oligomer is a dimer, or < 0.33 in case the oligomer is a trimer, etc. (in general, if such ratio is < 1/number of monomers in the oligomer), it shows that the potency of the cytotoxic mode of action of the organic cytotoxin oligomer of the present invention based on the inhibition of the corresponding protein phosphatases is enhanced compared to the references in a synergistic manner that goes beyond the increased number of toxins in the conjugate.

The results in Fig. 12 and Tab. 7 show that the IC50_OATP1B1 value and the IC50_OATP1B3 value of the MC-PrgTyr-Arg-Dimer-1 and MC-PrgTyr-Arg-Dimer-2 according to the invention were substantially increased in comparison to the reference microcystins. For IC50_OATP1B1, the increase was more than 116-fold for MC-PrgTyr-Arg-Dimer-1 and more than 175-fold for MC-PrgTyr-Arg-Dimer-2 in comparison to MC-LR, as well as 24-fold and more than 36-fold compared to the corresponding MC-PrgTyr-Arg monomer. A similar effect was also observed in case of IC50_OATP1B3 with a 134-fold increase for MC-PrgTyr-Arg-Dimer-1 and a 234-fold increase for MC-PrgTyr-Arg-Dimer-2 in comparison to MC-LR, as well as a 26-fold increase and a 45-fold increase, respectively, compared to the MC-PrgTyr-Arg monomer. This shows that the tested oligomers of the present invention have a significantly lowered general cytotoxicity on OATP1B1 and OATP1B3 expressing cell lines in comparison to the references.

On the other hand, however, the results clearly show that the protein phosphatase inhibition of the MC-PrgTyr-Arg-Dimer according to the present invention was significantly increased, as indicated by the more than 20-fold lower IC50_PPI for MC-PrgTyr-Arg-Dimer-1 and a more than 50-fold lower IC50_PPI for MC-PrgTyr-Arg-Dimer-2 compared to the IC50_PPI of the corresponding MC-PrgTyr-Arg monomer, as well as the about 10-fold lower IC50_PPI for MC-PrgTyr-Arg-Dimer-1 and the about 16-fold lower IC50_PPI for MC-PrgTyr-Arg-Dimer-2 compared to the IC50_PPI of the MC-LR reference cytotoxin.

Given that the general cytotoxicity on the OATP1B1 and OATP1B3 expressing cell lines of the MC-PrgTyr-Arg-Dimer according to the present invention is strongly decreased whilst its enzyme inhibitory effect is strongly increased, these results demonstrate that the oligomerization leads to the desired effect of greatly reduced active uptake into the cells.

In addition, the more than 20-fold to more than 50-fold lower IC50_PPI of the MC-PrgTyr-Arg-Dimers compared to the IC50_PPI of the corresponding MC-PrgTyr-Arg monomer are clear evidence of the synergistic effect of the oligomerization on the enzyme inhibition rather than just an additive effect from the accumulated individual cytotoxicity of the two monomers.

Finally, the more than 20-fold to more than 50-fold lower IC50_PPI in conjunction with the ratios of the general cytotoxicity on the OATP1B1 and OATP1B3 expressing cell lines and the protein phosphatase inhibition (as a measure of the PPI-normalized OATP uptake) show that with the MC-PrgTyr-Arg-Dimers according to the invention the therapeutic windows for both OATP-expressing cell lines was magnified by at least two orders of magnitude and up to four orders of magnitude in comparison to the MC-PrgTyr-Arg monomer (more than 700-fold to more than 2,400-fold) and the MC-LR reference cytotoxin (more than 1,000-fold to more than 3,900-fold).

It is understood that the results obtained with the two MC-PrgTyr-Arg-Dimers are representative of the beneficial effects of the organic cytotoxin oligomers of the present invention in general. Similar improvements and advantageous effects in terms of general cytotoxicity, protein phosphatase inhibition and/or therapeutic window are obtained with the other representative organic cytotoxin oligomers described in the other examples above.

These synergistic effects and improvements as well as the advantages associated therewith could not have been expected by a person skilled in the art.

Furthermore, it is understood that the invention is not limited to the detailedly described embodiments. Rather, the invention encompasses every new feature as well as every new combination of features, which in particular includes every combination of features in the patent claims and the description, even if this feature or this combination of features is not explicitly defined in the patent claims, the description or the embodiments.

For example, the skilled artisan understands that the microcystin dimers described in examples 2-21 can be also constructed with different microcystin analogues as monomers, either as homomeric microcystin dimers or as heteromeric microcystin dimers and/or with different bridging moieties, if any. In particular, such microcystin analogues can have a different amino acid sequence than those included in examples 2-21, e. g. microcystin analogues with different amino acids in position Aa¹, X², Aa³, Z⁴, and Aa⁷ and/or with different coupling functionalities such as an alkene, carboxyl, ketone, hydroxyl, hydroxylamine, isothiocyanate, tetrazine, thiol, aldehyde, for example. Furthermore, microcystin dimers can be also constructed by crosslinking the monomers in different positions, e. g. position Aa¹ of the first microcystin entity with position X² of the second microcystin entity or any other combination excluding position Aa⁵ and Aa⁶.

Likewise, microcystin trimers described in examples 26-33 can be also constructed with different microcystin analogues as monomers, either as homomeric microcystin trimers or as heteromeric microcystin trimers. In particular, such microcystin analogues can have a different amino acid sequence than those included in examples 26-33, e. g. microcystin analogues with different amino acids in position Aa¹, X², Aa³, Z⁴ and/or Aa⁷, and/or with different coupling functionalities such as an alkene, alkyl halide, hydrazide, carboxyl, ketone, hydroxyl, hydroxylamine, isothiocyanate, tetrazine, thiol, aldehyde, for example. The crosslinking domain can also covalently link different positions of the microcystin entities.

Furthermore, microcystin oligomers such as microcystin tetramers, microcystin pentamers, microcystin hexamers etc. can also be constructed, either as homomeric microcystin oligomers or as heteromeric microcystin oligomers, including, but not limited to, the microcystin entities used in examples 26-33 or microcystin analogues with different amino acids and/or different coupling functionalities, as noted above.

The nodularin dimers described in examples 34-39 can also be constructed with different nodularin analogues as monomers, either as homomeric nodularin dimers or as heteromeric nodularin dimers. In particular, such nodularin analogues can have a different amino acid sequence than those included in examples 34-39, e. g. nodularin analogues with different amino acids in position Aa¹, Aa² and/or Aa⁵, and/or with different coupling functionalities such as an alkene, carboxyl, ketone, hydroxyl, hydroxylamine, isothiocyanate, tetrazine, thiol, aldehyde, for example.

The nodularin trimers described in examples 40 and 41 can be also constructed with different nodularin analogues as monomers, either as homomeric nodularin trimers or as heteromeric nodularin trimers, in particular with nodularin analogues as cytotoxin entities which have a different amino acid sequence than those included in examples 34-41.

Furthermore, nodularin oligomers such as nodularin tetramers, nodularin pentamers, nodularin hexamers etc. can be also constructed, either as homomeric nodularin oligomers or as heteromeric nodularin oligomers, including, but not limited to, the nodularin entities used in examples 34-41 or nodularin entities with different amino acids and/or different coupling functionalities, as noted above.

The amatoxin dimers described in examples 42-47 can be also constructed with different amatoxin entities as monomers, either as homomeric amatoxin dimers or as heteromeric amatoxin dimers. In particular, the amatoxin entities can have a different amino acid sequence than those included in examples 42-47, e. g. amatoxin entities with different amino acids in position Aa¹, Aa³, Aa⁴, Aa⁵, Aa⁶ and/or Aa⁷ and/or with different coupling functionalities such as an alkene, carboxyl, ketone, hydroxyl, hydroxylamine, isothiocyanate, tetrazine, thiol, aldehyde, oxime.

Furthermore, the amatoxin trimers described in examples 48-51 can be also constructed with different amatoxin entities as monomers, either as homomeric amatoxin trimers or as heteromeric amatoxin trimers, in particular with amatoxin entities having a different amino acid sequence and/or different coupling functionalities than those representatively shown in the examples. Moreover, amatoxin oligomers such as amatoxin tetramers, amatoxin pentamers, amatoxin hexamers etc. can be also be constructed, either as homomeric amatoxin oligomers or as heteromeric amatoxin oligomers.

It is also understood that the anti-HER2 antibody trastuzumab used in examples 52-55 is purposively selected because trastuzumab is an established benchmark antibody for the genus of anti-TAA antibodies and anti-TSA antibodies. The results obtained with ADCs including trastuzumab thus demonstrate that the invention can also be practiced with other antibody species, in particular of the genus anti-TAA and anti-TSA antibodies.

Accordingly, it is also understood that the ADCs of examples 52-55 can also be constructed with different monoclonal antibodies that target different antigens, e. g. CD7 and BCMA, expressed by different cancer cells. Naturally, the ADCs can also be constructed with other homomeric and heteromeric microcystin, nodularin and/or amatoxin dimers and oligomers as already set out above, as well as with different linkers and using other conjugation methods, for example.

## Claims

1. A pharmaceutical cytotoxin oligomer, comprising
a first organic cytotoxin entity,
at least a second organic cytotoxin entity, and
a crosslinking entity forming a covalent linkage between the first organic cytotoxin entity and the second organic cytotoxin entity,
wherein a monomer of the first organic cytotoxin entity and the second organic cytotoxin entity exerts a cytotoxic effect on a target cell
i) by being actively transported into the target cell by a transporter protein of the target cell, and/or
ii) by inhibiting an intracellular enzyme of the target cell,
wherein the covalent linkage between the first organic cytotoxin entity and the second organic cytotoxin entity is substantially resistant to cleavage under physiological conditions.

2. The pharmaceutical cytotoxin oligomer according to claim 1, wherein the first organic cytotoxin entity and the second organic cytotoxin entity each comprises a cyclic oligopeptide with a plurality of amino acids, and
wherein the crosslinking entity covalently links a side-chain of one of the amino acids of the first organic cytotoxin entity with a side-chain of one of the amino acids of the second organic cytotoxin entity.

3. The pharmaceutical cytotoxin oligomer according to any one of claims 1 or 2, wherein the first organic cytotoxin entity and the second organic cytotoxin entity each comprises a microcystin having a general structure
cyclo (-Aa¹-X²-Aa³-Z⁴-Aa⁵-Aa⁶-Aa⁷),
wherein, independently from each other, Aa¹ and Aa³ each represent a D-amino acid, Aa⁵ is selected from the group consisting of Adda, DM-Adda, dm-Adda, (6Z)Adda, and ADM-Adda, Aa⁶ is selected from the group consisting of D-Glu and D-Glu(OCH₃), Aa⁷ is selected from the group consisting of Mdha, MdhB, Dha, L-Ser, L-MeSer, Dhb, (E)-Dhb, (Z)-Dhb, MeLan, Cys and Thr or a modified L-amino acid, and X² and Z⁴ are, independently from each other, an L-amino acid,
wherein the crosslinking part covalently links a side-chain of one of Aa¹, X², Aa³, Z⁴ or Aa⁷ of the first organic cytotoxin entity with a side-chain of one of Aa¹, X², Aa³, Z⁴ or Aa⁷ of the second organic cytotoxin entity.

4. The pharmaceutical cytotoxin oligomer according to any one of claims 1 or 2, wherein the first organic cytotoxin entity and the second organic cytotoxin entity each comprises a nodularin having a general structure
cyclo [-Aa¹-Aa²-Aa³-Aa⁴-Aa⁵],
wherein, independently from each other, Aa¹ is D-Asp or D-MeAsp or a modified D-amino acid, Aa² is Arg or Har or a modified L-amino acid, Aa³ is selected from the group consisting of Adda, DM-Adda, (6Z)Adda, and MeAdda, Aa⁴ is selected from the group consisting of D-Glu and D-Glu(OCH₃), Aa⁵ is Dhb or Mdhb or a modified L-amino acid, ,
wherein the crosslinking entity covalently links a side-chain of Aa¹, Aa² or Aa⁵ of the first organic cytotoxin entity with a side-chain of one of Aa¹, Aa² or Aa⁵ of the second organic cytotoxin entity.

5. The pharmaceutical cytotoxin oligomer according to any one of claims 1 or 2, wherein the first organic cytotoxin entity and the second organic cytotoxin entity each comprises an amatoxin having a general structure
cyclo {Aa¹-cyclo[Aa²-Aa³-Aa⁴-Aa⁵-Aa⁶]-Aa⁷-Aa⁸} (SEQ ID NO:1),
wherein, independently from each other, Aa¹ is Ile, Hil or Dhil, Aa² is Trp or Htp, Aa³ is Gly, Aa⁴ is Ile, Aa⁵ is Gly, Aa⁶ is Cys, Aa⁷ is Asn or Asp and Aa⁸ is Pro or Hyp, with cross-linking between Aa² and Aa⁶ via a sulfoxide (S=O) bridge,
wherein the crosslinking entity covalently links a side-chain of one of Aa¹, Aa² or Aa⁸ of the first organic cytotoxin entity with a side-chain of one of Aa¹, Aa² or Aa⁸ of the second organic cytotoxin entity.

6. The pharmaceutical cytotoxin oligomer according to any one of claims 1 to 5, wherein the crosslinking entity comprises, or consists of, a covalent linkage selected from the group consisting of an amide bond, a thioamide bond, an ether bond, a thioether bond, a covalent bond containing a triazole, a dihydropyridazine or a higher order amine, a carbamate bond, a thiocarbamate bond, an urea bond, a thiourea bond, a phosphate ester bond, a phosphamide bond, a sulfonamide bond, an oxime bond, or any combination thereof.

7. The pharmaceutical cytotoxin oligomer according to any one of claims 1 to 6, wherein the crosslinking entity comprises a bridging moiety that interconnects the first organic cytotoxin entity and the second organic cytotoxin entity with each other, wherein the bridging moiety is an organic molecule with a molecular size of from about 50 Da to about 1,000 Da.

8. The pharmaceutical cytotoxin oligomer according to any one of claims 1 to 7, wherein the crosslinking entity comprises a coupling functionality configured for covalent conjugation of the pharmaceutical cytotoxin oligomer to an antibody,
wherein the coupling functionality is selected from the group consisting of amino group, carboxyl group, hydroxyl group, azido group, alkyne group, alkene group, thiol group, aldehyde group, keto group, tetrazine group, and any combination thereof.

9. The pharmaceutical cytotoxin oligomer according to any one of claims 1 to 8, comprising at least a further third organic cytotoxin entity, wherein the crosslinking entity forms a covalent linkage between the first organic cytotoxin entity, the second organic cytotoxin entity and at least the further third organic cytotoxin entity,
wherein the covalent linkage between the first organic cytotoxin entity, the second organic cytotoxin entity and at least the further third organic cytotoxin entity is substantially resistant to cleavage under physiological conditions.

10. The pharmaceutical cytotoxin oligomer according to any one of claims 1 to 9, wherein the resistance to cleavage comprises resistance to enzymatic cleavage and resistance to acid-induced cleavage at least down to a pH value of pH 4.5.

11. The pharmaceutical cytotoxin oligomer according to any one of claims 1 to 10, wherein the intracellular enzyme of the target cell is a phosphatase or a polymerase.

12. A method for manufacturing a pharmaceutical cytotoxin oligomer according to any one of claims 1 to 11, comprising
A) providing a first organic cytotoxin entity and at least a second cytotoxin entity, wherein each of the first organic cytotoxin entity and the at least second organic cytotoxin entity exerts a cytotoxic effect on a target cell
i) by being actively transported into the target cell by a transporter protein of the target cell, and/or
ii) by inhibiting an intracellular enzyme of the target cell,
B) forming a covalent linkage between the first organic cytotoxin entity and the at least second organic cytotoxin entity that is substantially resistant to cleavage under physiological conditions, thereby forming the cytotoxin oligomer.

13. The method according to claim 12, wherein A) further comprises providing at least one organic bridging moiety, wherein the bridging moiety is an organic molecule with a molecular size of from about 50 Da to about 1,000 Da, and B) further comprises forming a first part of the covalent linkage by covalently conjugating the bridging moiety to the first organic cytotoxin entity, and forming at least a second part of the covalent linkage by covalently conjugating the bridging moiety to the at least second organic cytotoxin entity, thereby crosslinking the first organic cytotoxin entity and at least the second organic cytotoxin entity with each other via the bridging moiety.

14. An antibody-drug conjugate comprising
an antibody or an antigen-binding fragment thereof,
a pharmaceutical cytotoxin oligomer according to any one of claims 1 to 11,
wherein the pharmaceutical cytotoxin oligomer is covalently attached to the antibody or the antigen-binding fragment thereof.

15. The antibody-drug conjugate according to claim 14, having a general formula (I)
Ab- [L- (T)ₘ]ₙ (I)
wherein Ab is the antibody or antigen-binding fragment thereof; T is the pharmaceutical cytotoxin oligomer; L is an organic linker; and m is an integer from 1 to 5 and n is an integer from 1 to 8.

16. The antibody-drug conjugate according to any one of claims 14 or 15, wherein the linker is configured to release the pharmaceutical cytotoxin oligomer from the antibody or antigen-binding fragment thereof upon receptor-mediated endocytosis and/or lysosomal processing of the antibody-drug conjugate.

17. A method for manufacturing an antibody-drug conjugate, comprising
a) providing a pharmaceutical cytotoxin oligomer according to any one of claims 1 to 11,
b) providing an antibody or an antigen-binding fragment thereof,
c) covalently attaching the pharmaceutical cytotoxin oligomer to the antibody or the antigen-binding fragment thereof.

18. The method according to claim 17, wherein c) further comprises
c1) providing a linker,
c2) covalently attaching the pharmaceutical cytotoxin oligomer to the linker,
c3) covalently attaching the linker to the antibody or the antigen-binding fragment thereof.
